# EUROPEAN PATENT APPLICATION

(11) **EP 3 669 895 A1**
(43) Date of publication of application: **24.06.2020**
(21) Application number: 19207186.8
(22) Date of filing: 22.02.2008
(51) Int. Cl.: A61K 49/00, A61B 5/08

(54) **MEDICATION ADHERENCE MONITORING SYSTEM**

(30) Priority: 22.02.2007 US 891085 P
(62) Divisional of application: 08730539.7
(71) Applicant: University of Florida Research Foundation, Incorporated, Gainesville FL 32611 (US)
(72) Inventor: MELKER, Richard J., Newberry, Florida 32669 (US); DENNIS, Donn Michael, Gainesville, Florida 32610 (US); BATICH, Christopher, D., Gainesville, Florida 32606-6199 (US); GOLD, Mark, S., Alachua, Florida 32616 (US)
(74) Representative: Predazzi, Valentina

(57) **Abstract**

The present invention relates to the detection of markers in exhaled breath, wherein the detection of the presence or absence of the marker(s) in exhaled breath is used to assess various clinical data, including patient adherence in taking the medication and patient enzymatic (metabolic) competence in metabolizing the medication. An embodiment of the invention comprises a parent therapeutic agent labeled with a marker, where upon metabolism (e.g., via enzymatic action) of the therapeutic agent, the marker becomes volatile or semi-volatile and is present in the breath. In certain related embodiments, the marker contain a deuterium label, which is also present in the breath upon metabolism of the therapeutic agent. In another embodiment of the invention, the therapeutic agent is associated with a taggant (that may be either labeled or unlabeled with deuterium), which in turn will generate a marker in the breath that is easily measurable.

## Description

### FIELD OF INVENTION

The present invention relates to marker detection, in the form of odors or the like, to monitor medication adherence, and, more particularly, to a method and apparatus for the detection of markers in exhaled breath after the medication is taken by a patient, wherein such markers are combined with the medication.

### BACKGROUND INFORMATION

Breath is a unique bodily fluid. Unlike blood, urine, feces, saliva, sweat and other bodily fluids, it is available on a breath to breath and therefore continuous basis. It is readily available for sampling non-invasively Because the lung receives nearly 100% of the blood flow from the right side of the heart and has an anatomical structure (e.g., an alveolar-capillary membrane that is only 200-1000 nm thick and separates the blood from the gas in the lungs) that contains a massive surface area for effective diffusion of gases (e.g., transport oxygen and carbon dioxide), it has been suggested that the concentration of analytes/compounds in breath not only correlate with their blood concentrations, but will also rapidly change to sudden changes in analyte/compound concentrations in the blood. Other positive aspects of sampling the breath, as opposed to other bodily fluids, is that breath is less likely to be associated with the transfer of serious infections, is less intrusive to subjects who require the collection of biological samples (e.g., urine collection for drug testing), and is preferred by individuals who collect biological samples from subjects for health care assessments and/or drug testing (e.g., health care providers drawing blood or collecting urine, saliva and other non-breath biological media). Further, the collection of breath samples is relatively straightforward and painless.

The breath is comprised of two components: 1) a gas phase, and 2) a liquid phase. The liquid phase of breath is formed by aerosol droplets plus condensed water from the gas phase. Exhaled breath contains nearly 100% humidity at 37°C (body temperature). The aerosol droplets in exhaled breath are likely formed from the bulk flow of air over the airway lining fluid (ALF), which is a thin layer of liquid that lines a significant portion of the airway passages in the lung. The ALF can be considered an ultrafiltrate of blood, and allows transport of molecules from one side of the alveolar-capillary membrane to the other, either by 1) transmembrane passage (e.g., most uncharged, lipophilic molecular entities) and/or by transport through paracellular spaces located between cells that doesn't require transport directly through cell membranes (e.g., most charged and/or highly water soluble molecular entities). Therefore, not surprisingly, a wide variety of analytes/compounds with different properties (e.g., volatile, semi-volatile, non-volatile, hydrophobic, hydrophilic, charged, uncharged, small and large) that are in the blood can rapidly cross the capillary-alveolar and appear in the breath. If the temperature of the collected sample is maintained at 37°C or higher, volatile or semi-volatile analytes, particularly those that are relatively insoluble in water and readily diffuse out of water, will preferentially remain in the gas state of breath and can be treated as a gas for compounds. In this instance, sensors designed to work with gaseous media would be preferable. For compounds that are highly water soluble and likely to remain in solution, the exhaled breath sample can be collected as a condensate when cooled. This liquid can then be analyzed with sensors that are designed for liquid-based analyses. Compounds likely to be detectable in the gas phase typically are lipophilic (hydrophobic) and semi-volatile or volatile molecules such as the intravenous anesthetic agent, propofol (vapor pressure ∼ 0.2 mmHg at 37 °C), while compounds likely to be detected in the liquid phase are hydrophilic, non-volatile and/or significantly charged at physiological pH (normal pH =7.4), such as glucose, lactic acid, most therapeutic agents and electrolytes (e.g., Cl⁻, Na⁺, K⁺, Ca²⁺, Mg⁺²) . Thus an exhaled breath sample can be handled to produce a gaseous matrix for certain compounds and sensors, and a liquid matrix for others. In instances where it is desirable to detect more than one compound (*e.g.,* detection of hydrophilic and hydrophobic molecules in the breath), the sample can be split and a portion maintained as a gas and a portion condensed as a liquid.

Medication non-compliance (or non-adherence) is the failure to take drugs on time in the dosages prescribed, which results in patient undermedication or overmedication. Lack of medication adherence is as dangerous and costly as many illnesses. As any physician or caregiver understands, medicine is only effective when taken as directed.

Noncompliance cuts across all categories of patients and illnesses. People with breast cancer, organ transplants, and hypertension, as well as people on a short course of antibiotics, can all forget to take their medications. Researchers have identified more than 200 variables that affect whether a patient will be compliant. Compliance rates are also likely to decline over time, especially for patients with asymptomatic diseases.

Non-compliance of patients to drug regimens prescribed by their physicians results in excessive healthcare costs estimated to be around $100 billion per year through lost work days, increased cost of medical care, higher complication rates, as well as drug wastage. Studies have shown that non-compliance causes 125,000 deaths annually in the U.S. alone [Smith, D., "Compliance Packaging: A Patient Education Tool," American Pharmacy, NS29(2) (1989)]. Moreover, medication non-adherence leads to 10 to 25 percent of hospital and nursing home admissions, and is becoming an international epidemic [Standberg, L.R., "Drugs as a Reason for Nursing Home Admissions," American Healthcare Association Journal, 10(20) (1984); Schering Report IX, The Forgetful Patient: The High Cost of Improper Patient Compliance; Oregon Department of Human Resources, A study of Long-Term Care in Oregon with Emphasis on the Elderly, (March 1981)].

About 50% of the 2 billion prescriptions filled each year are not taken correctly [National Council for Patient Information and Education]. 1/3 of patients take all their medicine, 1/3 or patients take some dosage of the prescribed medicine, 1/3 of patients do not take any at all [Hayes, R.B., NCPIE Prescription Month, (October 1989)]. Such sub-optimal rates of compliance reported by various studies becomes of even greater concern as the American populace ages and becomes more dependent on drugs to fight the illnesses accompanying old age. By 2025, over 17% of the US population will be over 65 [Bell JA, May FE, Stewart RB: Clinical research in the elderly: Ethical and methodological considerations. Drug Intelligence and Clinical Pharmacy, 21: 1002-1007, 1987] and senior citizens take, on average, over three times as many drugs compared to the under 65 population [Cosgrove R: Understanding drug abuse in the elderly. Midwife, Health Visitor & Community Nursing 24(6):222-223, 1988]. The forgetfulness that sometimes accompanies old age also makes it even more urgent to devise cost-effective methods of monitoring compliance on a large scale.

Further, non-compliance of patients with communicable diseases costs the public health authorities millions of dollars annually and increases the likelihood of drug-resistance, with the potential for widespread dissemination of drug-resistant pathogens resulting in epidemics. For example, one of the most serious consequences of noncompliance involves the outbreaks of new, drug-resistant strains of HIV and tuberculosis (TB), which have been significantly attributed to patients who do not properly follow their complex medication regimens. In addition, the long-term misuse of antibiotics has given rise to forms of previously treatable diseases that are impervious to the most advanced medications.

Current methods of improving medication adherence for health problems are mostly complex, labor-intensive, and not predictably effective [McDonald, HP et al., "Interventions to enhance patient adherence to medication prescriptions: scientific review," JAMA, 289(4):3242 (2003)]. A cost-effective, but difficult to administer, program has been developed in seven locations around the nation to combat this serious threat to the American populace. It involves direct observation of all drug delivery by trained professionals (directly observed therapy: DOT) but is impractical for large scale implementation. Many techniques are also invasive, *e.g.,* blood sampling.

Previous medication adherence monitoring systems disclosed by the present inventors related to the use of exhaled breath as a means to detect when and/or whether a subject has taken medication as prescribed (see, for example, U.S. Patent Application Serial Nos. 10/722,620 (filed November 26, 2003) and 11/097,647 (filed April 1, 2005)). The monitoring systems described in those applications either detected in exhaled breath the medication; a metabolite of the medication; or a detectable marker (that was combined with the medication) or its metabolite. Many of the markers considered for use in those applications were largely GRAS ("Generally Recognized As Safe") compounds, as classified by the FDA. Unfortunately, currently available detectors (sensors) do not detect these compounds in exhaled breath reliably (e.g., issues due to sensitivity or discrimination from potential interferents) to be used in practical devices for many medication adherence applications.

Accordingly, there is a need in the art for a system and method to improve drug compliance which provides simple monitoring of medication dosing which is non-invasive, intuitive and sanitary. In particular, there is a need for a unique group of markers that can be combined with medications for adherence monitoring, where the markers are sufficiently volatile to be detected in the gas phase of exhaled breath, even at very low concentrations using current detection technologies.

### SUMMARY OF THE INVENTION

The present invention solves the needs in the art by providing systems and methods for non-invasive monitoring of medication adherence by detecting a marker in exhaled breath that is the product of medication absorption, distribution, metabolism, and/or excretion in the patient's body.

### BRIEF DESCRIPTION OF THE FIGURES

For a more complete understanding of the present invention, and the advantages thereof, reference is now made to the following descriptions taken in conjunction with the accompanying drawings, in which:
Figures 1-59 illustrate various aspects of the invention relating to the use of isotopic labels as detectable markers for monitoring patient medication adherence.
**Figure 1** shows hydrolysis reactions of the esterase type - carboxylic ester hydrolases (EC 3.1.1).
**Figure 2** show illustrative examples of select alcohols and their physiochemical properties.
**Figure 3** show illustrative examples of carboxylic acids and their physicochemical properties.
**Figure 4** show dealkylation reactions by CYP450 (Example: Demethylation).
**Figure 5** show physicochemical and toxological properties of select aldehydes.
**Figure 6A** shows metabolic fate of selected ordinary isotope-labeled alcohols, aldehydes, and carboxylic acids..
**Figure 6B** shows a non-ordinary isotope-labeled alcohols, aldehydes and carboxylic acids.
**Figure 7A** shows illustrative examples of therapeutic agents undergoing desmethylation and generating formaldehyde.
**Figure 7B** shows illustrative examples of therapeutic agents undergoing desmethylation and generating formaldehyde.
**Figure 8** shows illustrative examples of therapeutic agents undergoing desethylation and generating acetaldehyde.
**Figure 9** shows illustrative examples of therapeutic agents undergoing despropylation and generating propionaldehyde.
**Figure 10A-B** show illustrative examples of therapeutic agents undergoing desbutylation and generating butyraldehyde, including an illustration of butyraldehyde.
**Figure 11** shows a gas phase FTIR-based absorption spectrum of human breath.
**Figure 12** shows a gas phase FTIR-based absorption spectrum of ethanol in nitrogen gas.
**Figure 13** shows a gas phase FTIR-based absorption spectrum of d5 ethanol in nitrogen gas.
**Figure 14** shows a gas phase FTIR-based absorption spectrum of d2 ethanol in nitrogen gas.
**Figure 15** shows a gas phase FTIR-based absorption spectrum of ethanol, d5 ethanol, and d2 ethanol in nitrogen gas.
**Figure 16** shows a gas phase FTIR-based absorption spectrum of methanol, ethanol, d3 methanol, and d5 ethanol in nitrogen gas.
**Figure 17** shows a gas phase FTIR-based absorption spectrum of d2 ethanol in human breath.
**Figure 18** shows a gas phase FTIR-based absorption spectrum of d2 ethanol in human breath with background breath absorption substracted.
**Figure 19** shows a gas phase FTIR-based absorption spectrum of acetaldehyde and d4 acetaldehyde in nitrogen gas.
**Figure 20** shows a gas phase FTIR-based absorption spectrum of d5 ethanol and d4 acetaldehyde in nitrogen gas.
**Figure 21** shows a gas phase FTIR-based absorption spectrum of d5 ethanol and d4 acetaldehyde in human breath.
**Figure 22** shows a gas phase FTIR-based absorption spectrum of benzene (C**₆**H**₆**), ¹³C-labeled benzene (¹³C**₆**H**₆**), and deuterated benzene (C**₆**D**₆**) in nitrogen gas.
**Figure 23** shows a gas phase FTIR-based absorption spectrum of acetaldehyde and ¹³C-labeled acetaldehyde (¹³CH₃¹³CHO) in nitrogen gas.
**Figure 24** shows a gas phase FTIR-based absorption spectrum of formaldehyde and d2 formaldehyde in nitrogen gas.
**Figure 25** shows a gas phase FTIR-based absorption spectrum of acetaldehyde and d4 acetaldehyde in nitrogen gas.
**Figure 26** shows a proposed FTIR deuterium labeled spectral monitoring bands to distinguish deuterated ethanol, deuterated acetaldehyde, and deuterated benzene in nitrogen gas.
**Figure 27** shows proposed FTIR deuterium labeled spectral monitoring bands to distinguish d3 methanol, d5 ethanol, d4 acetaldehyde, d6 benzene, and d8 styrene in Nitrogen gas.
**Figure 28** shows illustrative examples of amines that appear in food.
**Figure 29** shows an ester example of a GRAS agent listed as food additive (Class 1 Drug) - Aspartame: An ester food additive metabolized by human gut esterases and gut peptidases.
**Figure 30** shows an esterase example of a FDA Approved Drug (Class 2 Drug) - Aspirin (Acetylsalicylic Acid): An ester drug metabolized by aspirin esterases in humans.
**Figure 31** shows an ester example of GRAS agents listed As food additives (Class 1 Drugs) - methyl, ethyl, propyl and butyl parabens: ester food additives metabolized by human carboxylesterases and tissue esterases.
**Figure 32** shows an esterase example of a FDA approved drug (Class 2 Drug) - Clofibrate: An ester drug metabolized by esterases in humans.
**Figure 33** shows an esterase example of a FDA approved drug (Class 2 Drug) - Esmolol: A drug metabolized by arylesterase located within the cytosol of human red blood cells.
**Figure 34** shows an example of an ester FDA Approved Drug (Class 2 Drug) - Procaine: An ester drug metabolized by pseudocholinesterase (butyrylcholinesterase) located within human blood.
**Figure 35** shows an example of an esterase creation of new chemical entity (NCE) (Class 3 Drug) - Cyclic Structure Containing Three Ester Groups.
**Figure 36** shows an example of an esterase creation of new chemical entity (NCE) (Class 3 Drug) - Linear structure containing three ester groups.
**Figure 37** shows an example of an esterase creation of new chemical entity (NCE) (Class 3 Drug) - Linear structure containing four ester groups.
**Figure 38** CYP450 Example 1 - CYP-3A4-mediated Metabolism FDA Approved Drug (Class 2 Drug): Verapamil - An L-type Calcium Channel Blocker.
**Figure 39** shows CYP450 Example 2 - CYP-3A4-mediated Metabolism FDA Approved Drug (Class 2 Drug): Erythromycin - An Antibiotic.
**Figure 40** shows CYP450 Example 3 - CYP-3A4-mediated Metabolism FDA Approved Drug (Class 2 Drug): Amiodarone - An Antiarrhythmic Drug.
**Figure 41** shows CYP450 Example 4 - CYP-3A4-mediated Metabolism FDA Approved Drug (Class 2 Drug): Propafenone - An Antiarrhythmic Drug.
**Figure 42** CYP450 Example 5 - CYP-3A4-mediated Metabolism FDA Approved Drug (Class 2 Drug): Diltiazem - An Antiarrhythmic Drug.
**Figure 43** CYP450 Example 6 - CYP-2D6-mediated Metabolism FDA Approved Drug (Class 2 Drug): Flecainide - An Antiarrhythmic Drug.
**Figure 44** shows CYP450 Example 7 - CYP-2D6-mediated Metabolism FDA Approved Drug (Class 2 Drug): Codeine - A Prodrug Narcotic for Analgesia.
**Figure 45** shows CYP450 Example 8 - CYP-1A2-mediated Metabolism FDA Approved Drug (Class 2 Drug): Olanzapine - An Antipsychotic Agent.
**Figure 46** shows CYP450 Example 9 - CYP-1A2-mediated Metabolism Class 1 Drug: Caffeine - A Food Additive.
**Figure 47** shows CYP450 Example 10 - CYP-2C-mediated Deamination FDA Approved Drug (Class 2 Drug): Amphetamine - A CNS Stimulant.
**Figure 48** shows Deamination Example 1 - Adenosine Deaminase (EC 3.5.4.4) mediated Deamination FDA Approved Drug (Class 2 Drug): Adenosine - An Antiarrhythmic Agent.
**Figure 49A-C** shows MAMS: Illustrative Examples - Simple Approaches.
**Figure 50A-B** shows MAMS: Illustrative Examples - Approaches of Intermediate Complexity.
**Figure 51A-B** shows MAMS: Illustrative Examples - Approaches of Intermediate Complexity.
**Figure 52** shows MAMS: Illustrative Examples - Complex Approaches.
**Figure 53** shows MAMS: Illustrative Examples - Complex Approaches.
**Figure 54** shows MAMS: Illustrative Examples - Complex Approaches.
**Figure 55** shows MAMS: Illustrative Examples - Complex Approaches.
**Figure 56A-D** shows a hypothetical example - an illustration of how MAMS-11 would function.
**Figure 57A-C** shows MAMS: illustrative examples - one drug with many doses, Type 1.
**Figure 58A-C** shows MAMS: illustrative examples - one drug - many doses, Type 2.
**Figure 59A-C** shows MAMS: illustrative examples - one drug - many doses, Type 3.

### BRIEF DESCRIPTION OF THE APPENDICES

Also attached and made a part of this application is Appendix A, which provides Tables 1-23 with detailed listings of various aspects of the invention. These Appendices are incorporated by reference in this application in their entireties to the same extent as if fully set forth herein.

### DETAILED DESCRIPTION

The means to practice medication adherence monitoring in accordance with the subject invention are based on the development and use of markers as categorized below:
***Class 1***: Generally recognized as safe (GRAS) compounds including but not limited to food additives/components in industrialized countries and agents listed on the FDA inactive ingredient database;
***Class 2:*** Drugs already approved by governmental regulatory authorities (e.g., FDA) as therapeutic agents;
***Class 3:*** New chemical entities (NCEs) including those from slightly modified derivatives of Class 2 agents (e.g., chemically modified verapamil or dextromethorphan) or those derived from completely new chemical scaffolds (e.g., fluoroesters).

There are a variety of strategies for using Class 1, 2 and 3 marker compounds to assess whether a subject took his/her medication as prescribed by their health care provider. Central to all of these approaches is generation of a volatile (including semi-volatile or poorly volatile) chemical marker that appears in body fluids, preferably in the breath, hereafter termed the exhaled drug ingestion marker (EDIM). EDIMs arise from a number of sources or combination of sources, including:
***EDIM Source 1:*** any component of the active therapeutic drug matrix. The active therapeutic drug matrix contains three different components: 1) the active drug itself (Source **1_{A}**), 2) any associated salts (Source **1_{S}**) linked to the active drug (e.g., mesylate, acetate, tartrate, succinate, etc.), and 3) excipients (Source **1_{E}**).
***EDIM Source 2:*** metabolite(s) of the active therapeutic drug matrix, including active drug (Source **2_{A}**), salt (Source **2_{S}**), and/or excipients (Source **2_{E}**).
***EDIM Source 3:*** a compound that is associated with the active therapeutic drug matrix but is not an integral part of it *per se,* hereafter termed a taggant (i.e., the taggant is physically located adjacent but not physically integrated into the active therapeutic drug matrix).
***EDIM Source 4:*** a metabolite of the taggant.

Thus, according to the subject application, an EDIM could be liberated from as many as 8 general sources within a therapeutic (such as a pill) system. However, the exact number of EDIM sources in a given therapeutic system will be dependent upon other factors, including whether more than one excipient of the active therapeutic drug matrix is used as an EDIM source (usually more than one excipient is added to the final formulation) and/or whether more than one taggant is added to the pill system as EDIM sources, either from themselves or their metabolites. Similarly, since a number of therapeutic pills contain more than one active therapeutic agent (combination therapy) such as the anti-cholesterol agent Vytorin (ezetimibe and simvastatin), additional EDIM sources may arise from them or their metabolites.

For example, if the EDIM is a metabolite, it is most likely generated by enzymatic degradation but could also occur by spontaneous breakdown of compounds in the human body independent of specific enzyme action. The enzyme metabolism of many Class 1 through 3 agents to volatile (including semi-volatile or poorly volatile) EDIMs in the breath is well known, predictable or easily tested in various *in vitro* and *in vivo* systems. Humans contain a great variety of enzymes (Table 1) that can generate potential EDIMs. Examples include esterase-mediated degradation of esters (e.g., fluoroesters → fluoroalcohols + carboxylic acids) to their corresponding alcohols and carboxylic acids, alcohol dehydrogenase mediated degradation of 1° and 2° alcohols to their respective aldehydes and ketones, respectively; or CYP-mediated reactions via reduction, oxidation and/or hydrolysis to generate volatile (including semi-volatile or poorly volatile) metabolites (e.g., aldehydes via CYP-mediated dealkylation reactions). These will be discussed in greater detail below with specific examples illustrated. As shown above, different combinations of compounds (drug class 1, 2 and/or 3), which are enzymatically degraded by a wide variety of enzymes (e.g., esterases, dehydrogenases, CYP-450 fractions, deaminases), can be incorporated into a pill system that can potentially generates 8 different general EDIM sources (1_{A}, 1_{S}, 1_{E}, 2_{A}, 2_{S}, 2_{E}, 3, and 4). This architecture of the pill system allows the construction of several types of highly flexible, adaptable medication adherence monitoring system (MAMS) configurations.

The EDIM is a molecular entity that could either be naturally found in the body (endogenous) but be detected at concentrations that readily distinguish it from natural background breath levels for the MAMS application, or is unique (not endogenous to the human body) and can very easily distinguished in human breath. With regard to the latter, the incorporation of an isotope(s) into the EDIM or the compound (Class 1, 2 and/or 3 agent) that generates the EDIM, particularly those that are stable (non-radioactive) and not ordinary (i.e., not the most abundant form of atom found in nature) to a specific atom (e.g., deuterium at the hydrogen atom) to MAMS chemistry offers a multitude of major advantages. These include excellent chemistry flexibility (multiple isotopic labeling targets on various molecules), outstanding signal-to-noise ratio (labeled EDIMs would be easily distinguished against endogenous compounds that are not labeled with non-ordinary isotopes such as deuterium, which likely removes the need for baseline breath sampling), excellent safety in humans (selective isotopic labeling does not significantly alter the physiochemical/molecular properties of the molecule), and minimal-to-no effects on pharmacokinetics (absorption, distribution, metabolism, elimination [ADME]/pharmacokinetics [PK]) or pharmacodynamics (PD) of the active drug. These characteristics of an isotope-based MAMS indicate it would be able to navigate the regulatory "waters" (pathways) much more easily, be less complex, and be less expensive but yet be more reliable in its use and have a shorter time to market.

With regard to isotope-labeled EDIMs, the need for a baseline sample is predicated on the incidence of the non-ordinary isotope in nature (Table 2). Since the incidence of deuterium (²H) among H isotopes (0.015%) and ¹⁷O among O isotopes (0.037%) in nature is very low, the baseline sampling requirement here would be minimal compared to that of ¹³C among C isotopes (1.11%). In other words, the chances of isotopic background noise (non-ordinary isotopes that appear normally in nature and in the body) interfering with an isotopic-labeled EDIM measurement is 74-fold (=1.11%/0.015%) or 30-fold (=1.11%/0.037%) greater for a ¹³C-based label than for a deuterium-based or ¹⁷O-based EDIM labels, respectively. For these reasons, a preferred isotopic label for the current invention is deuterium. The EDIM-based isotopic (non-ordinary isotope) labels suitable for biological applications include but are not limited to those shown in Table 2. Similar to the case of non-isotopic (or known as ordinary isotopes) labeled EDIMs, isotopic labeled EDIMs may or may not require the action of enzymes (Table 1). Hereafter, the term labeled (e.g., labeled compound or labeled EDIM) or isotope in this application denotes the use of a "non-ordinary" isotope of an atom.

Current methods for adapting isotopes (both non-radioactive and radioactive) in clinical medicine can be applied to the subject invention. For example, breath tests using ¹³C, ¹⁴C to assess enzyme function, using ¹⁵N to improve the quality of biochemical tracer studies, or using ¹⁷O (for MRI scans) or ¹⁸O (for PET scans) to improve the quality of imaging can be applied to the subject invention.

For example, breath tests that require the oral or intravenous administration of ¹³C-labeled caffeine (Park-GJH et al, Hepatology 38:1227-1236, 2003) and intravenous administration of ¹⁴C-labeled erythromycin (US Patent 5,100,779) can be used to assess medication adherence by measuring the amount of carbon isotope-labeled carbon dioxide in the breath produced at various times or a fixed time post administration of a labeled drug/therapeutic of the invention. Table 2 depicts a variety of biologically relevant isotopes that could be useful for MAMS. By illustrating how various isotopic labels, preferably those that are stable (non-radioactive) including deuterium (²H), can be incorporated into the EDIM (EIDM Sources 1 [1_{A}, 1_{S}, 1_{E}], 2 [2_{A}, 2_{S}, 2_{E}], 3, and 4) and adding new embodiments, the current application will further teach and expand on the principle of applying isotopic labeling to medication adherence monitoring systems (MAMS).

### Overview of MAMS using isotopes

According to the subject application, isotope-based MAMS preferably contain 3 elements:
**Element 1** - medical chemistry: targeted, stable isotopic labeling, ideally with non-radioactive isotopes shown in Table 1 (most preferably with deuterium) of Class 1, 2 and/or 3 compounds that provide EDIM(s) from EDIM Sources 1 (1_{A}, 1_{S}, 1_{E}), 2 (2_{A}, 2s, 2_{E}), 3, and/or 4. In another embodiment, to maximize the number of different active therapeutic drugs that could be identified with MAMS, combinations of isotopic (e.g., deuterium)-labeled EDIMs could be combined in various ways with non-isotopic (e.g., ordinary hydrogen) EDIMs. The reviewer is referred below to the discussion in Element 2 for additional details on how isotopic chemistry will be used for MAMs.
**Element 2** - sensor: a variety of sensors modalities to measure the EDIM in breath were previously discussed in the prior patent applications listed in Introduction (Section A). According to the subject invention, preferred sensor embodiments include those that have the ability to measure isotopic-based EDIMs, such as gas chromatography detectors coupled to infrared-based detectors or gas chromatography mass spectroscopy sensors. More preferably, the sensor embodiments can comprise modified and optimized commercial off-the shelf (COTS) miniature gas chromatography (mGC) detector coupled to infrared (IR, liquid based for detection of poorly volatile isotopic-labeled EDIMs and/or gas based for detection of volatile or semi-volatile EDIMs) detection capabilities. This sensor will allow chromatographic separation of various EDIMs while simultaneously exploiting the powerful infrared (IR) spectroscopy (difference in mass among molecules containing isotopes have different vibrational modes) analytical capabilities to distinguish endogenous analytes from isotopic (preferably deuterated) ones. Alternately, a portable GC-MS would be suitable. Portable GC-MS could distinguish various isotopic labels (since isotopes have different masses) and thus greatly increase the number of taggants.

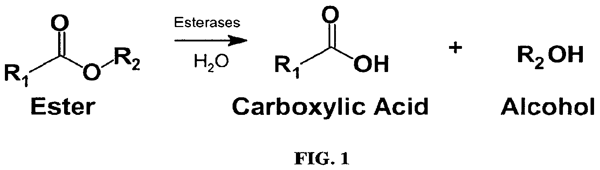

In contrast, IR alone would unlikely be able to discriminate between deuterated compounds of a given chemical class such as aliphatic alcohols (e.g., methanol versus ethanol) or aldehydes (e.g., formaldehyde versus acetaldehyde). The study of alcohols is important because a number of chemical classes, including but not limited to esters, carbonate esters, acetals, or ketals may be the optimal sources (e.g., chemistry flexibility, safety, etc.) for generating EDIMs with ideal characteristics for MAMS applications. As shown in Figure 1, an ester is hydrolyzed to its corresponding alcohol and carboxylic acid. Other chemical classes that can also enzymatically or spontaneously create alcohols include carbonate esters, acetals and ketals. Esters, carbonate esters, acetals and ketals can belong to Class 1, 2 or 3 (see Section A for classification).

In the embodiment illustrated in Figure 1, depending upon the ester, the EDIM(s) could be 1) an isotopic-labeled ester, 2) an isotopic-labeled alcohol derived from the isotopic-labeled ester, 3) an isotopic-labeled acid derived from the isotopic-labeled ester, and 4) an isotopic-labeled aldehyde or ketone derived from an isotopic-labeled 1° or 2° alcohol, which may or may not be generated from 1° or 2° alcohol-based esters, respectively. In addition, various combinations of isotopic-labeled esters and their associated labeled acids, labeled alcohols and/or labeled aldehydes/ketones could be used to provide unique EDIM signatures in the breath. The type of R2 group in the ester can be varied to sterically/electronically alter the susceptibility of the ester to hydrolysis, and will thus play a significant role in the rate of appearance of ester-derived labeled EDIM(s). The physicochemical properties (e.g., physical state, volatility) of the ester will be a function of both R1 and R2. By incorporating various isotopic labels listed in Table 2 (preferred embodiment is deuterium), where appropriate, into the various atomic sites of the esters, various EDIMs (arising from the ester, acid, alcohol and/or aldehyde/ketone) containing one or more isotopic labels could be generated that will fulfill the requirements of an effective MAMS.

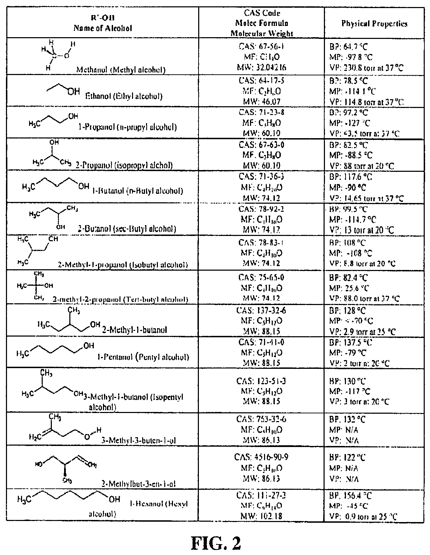

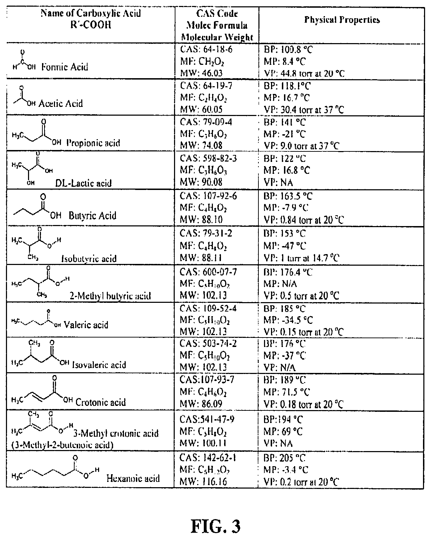

As shown in Table 2, a number of isotopes can be placed on key parts of ester molecules to liberate products (alcohols and/or acids) via ester hydrolysis that contain distinctive isotopic tags. The structures and key physicochemical characteristics of relevant alcohols and acids that may serve as isotopic-labeled EDIMs for MAMS are shown in tables in Figures 2 and 3, respectively. Figure 2 illustrates examples of select alcohols and their physiochemical properties. Figure 3 shows different carboxylic acids that are commonly generated via enzymatic degradation of GRAS-type flavoring additives and/or FDA approved drugs (e.g., esterase mediated degradation of esters to their corresponding acids and alcohols). Furthermore, as stated previously, the 1° and 2° alcohols will in turn be further metabolized by alcohol dehydrogenase to yield aldehydes and ketones, respectively. In an identical manner to that described above for esters, similar compounds, including but not limited to carbonate esters, acetals, and ketals could be labeled to generate alcohols (and subsequent generation of aldehydes/ketones) and/or carboxylic acids.

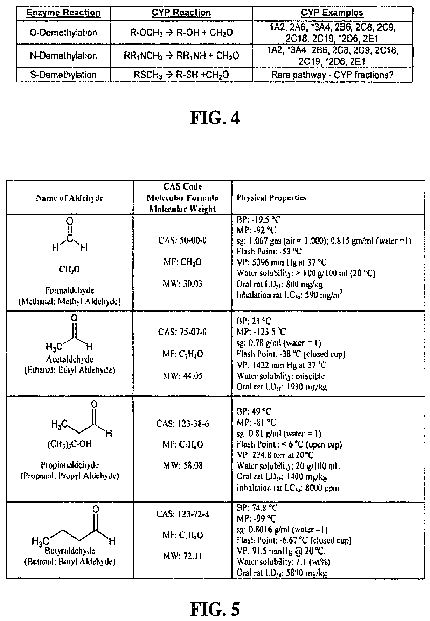

The study of aldehydes is important because the CYP450 enzyme system, which is by far the most important enzyme system for degrading drugs in humans predominantly via the processes of reduction, oxidation and hydrolysis, frequently generate different aldehydes via various types of dealkylation reactions. Among the different types of dealkylation reactions (Figure 4), demethylation (desmethyl metabolites via O-, N- and S-demethylation), which produces formaldehyde, is most notable. However, other important dealkylation reactions in human drug metabolism include deethylation (desethyl metabolites), depropylation (despropyl metabolites) and debutylation (desbutyl metabolites) reactions that generate acetaldehyde, propionaldehyde and butyraldehyde, respectively. The CYP450 substrates can belong to Class 1, 2 or 3 (see Section A for classification).

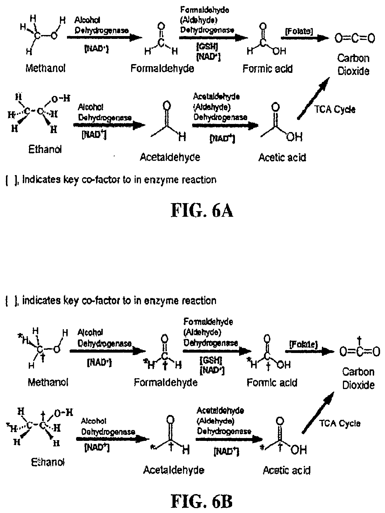

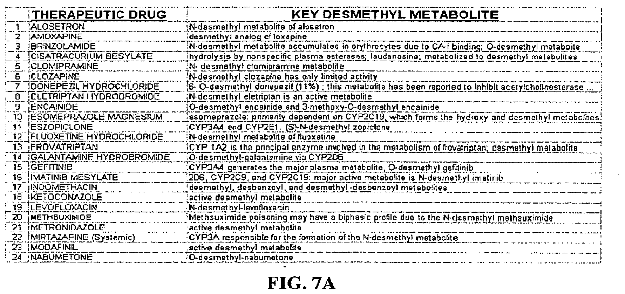

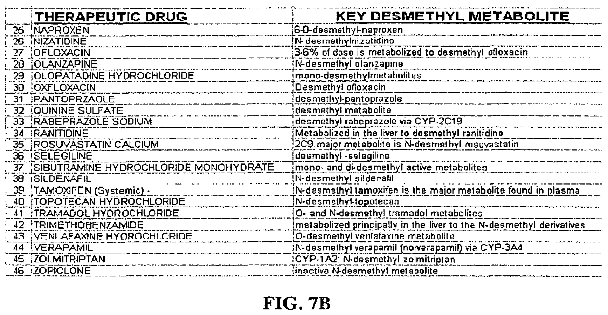

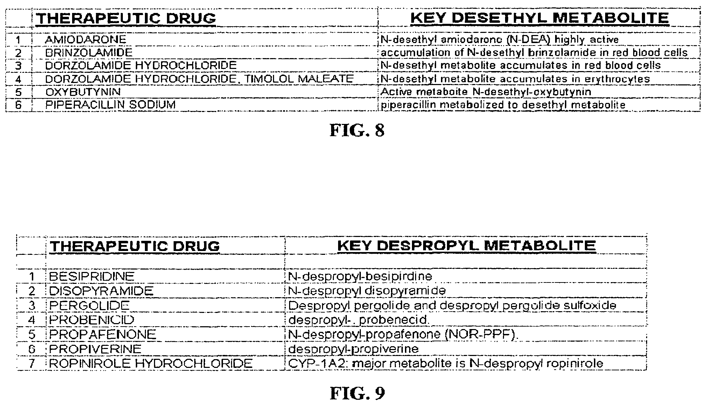

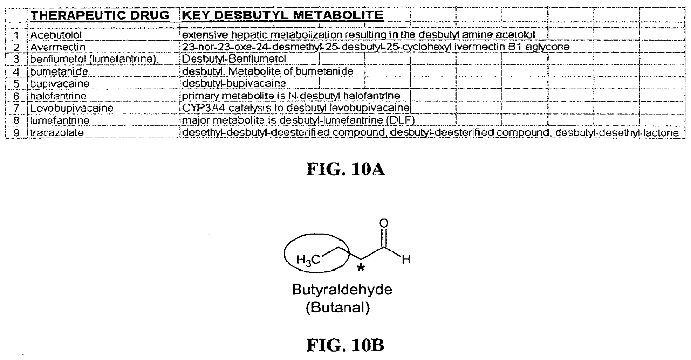

The structures and key physicochemical characteristics of some relevant aldehydes that may serve as isotopic-labeled EDIMs for MAMS are shown in Figure 5. Further metabolism of the primary alcohols, acids, and aldehydes discussed above is via the tricarboxylic acid (TCA) cycle, which will ultimately produce carbon dioxide and water as illustrated in the reaction scheme of Figure 6. On the other hand, shorter branched-chain aliphatic alcohols, aldehydes, and acids undergo beta-oxidative cleavage to yield intermediates of the amino acid and/or fatty acid metabolic pathways, which undergo subsequent complete metabolism to CO₂ and water via the tricarboxylic acid cycle. As chain length and substitution increase, the alcohols and aldehydes undergo a combination of omega-, omega-1 and beta-oxidation, and selective dehydrogenation and hydration to yield polar acidic metabolites. Numerous specific illustrative examples of how Class 1, 2 and/or 3 agents, which are degraded by different enzyme systems, can be used as isotope-labeled EDIMs *per se* or to liberate isotopic-labeled EDIMs for an effective MAMS will be shown in Section C. Examples of FDA-approved drugs that generate formaldehyde (Figure 7), acetaldehyde (Figure 8), propionaldehyde (Figure 9) and butyraldehyde (Figure 10) via CYP450-mediated desmethylation, desethylation, despropylation, and desbutylation, respectively, are illustrated.

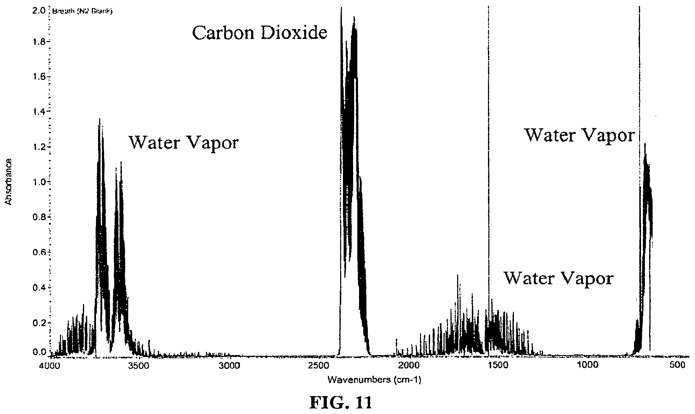

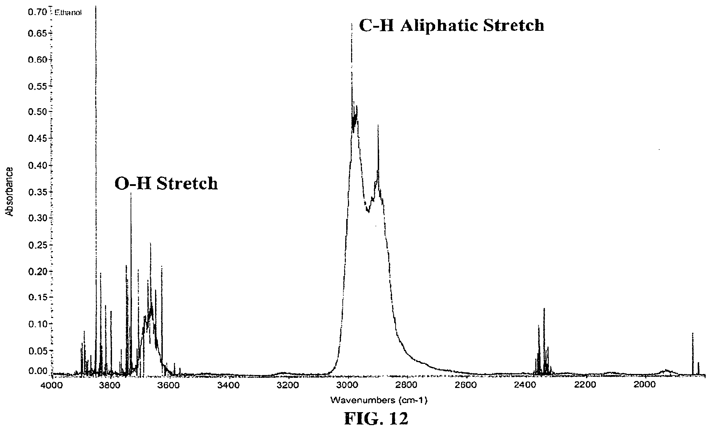

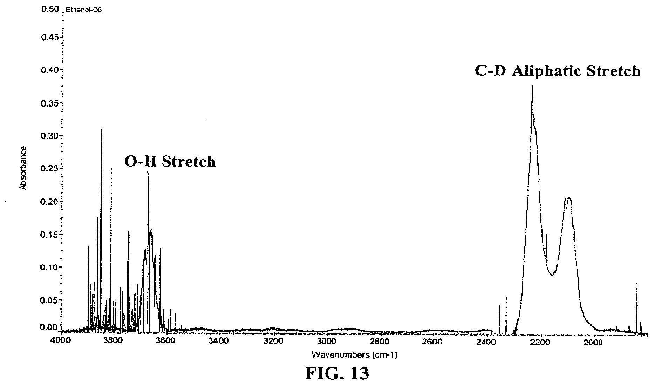

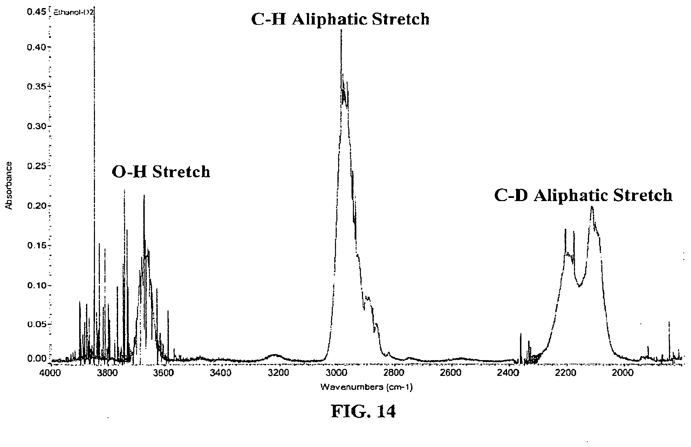

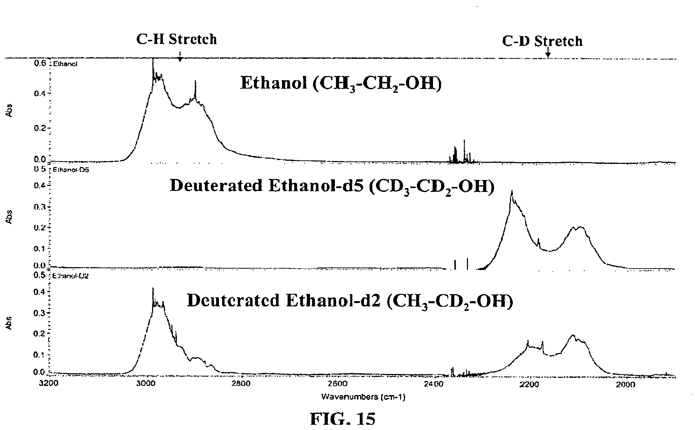

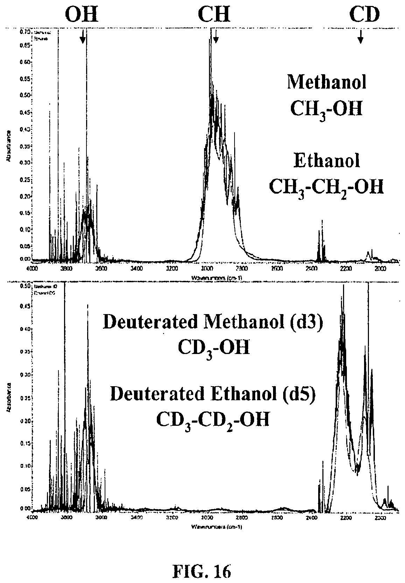

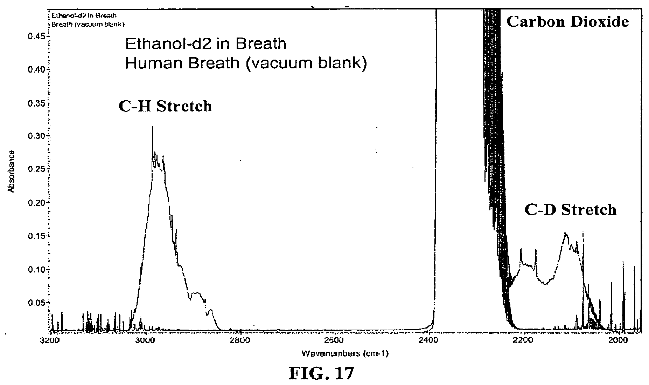

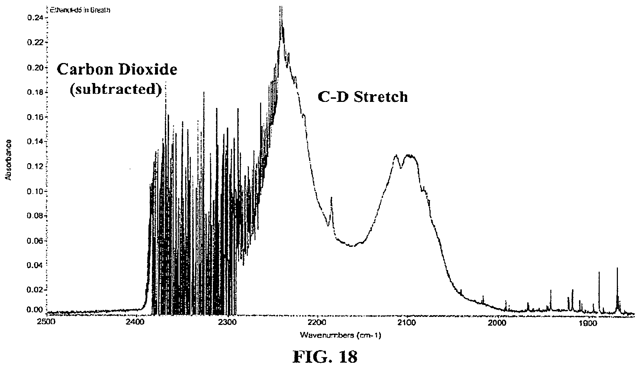

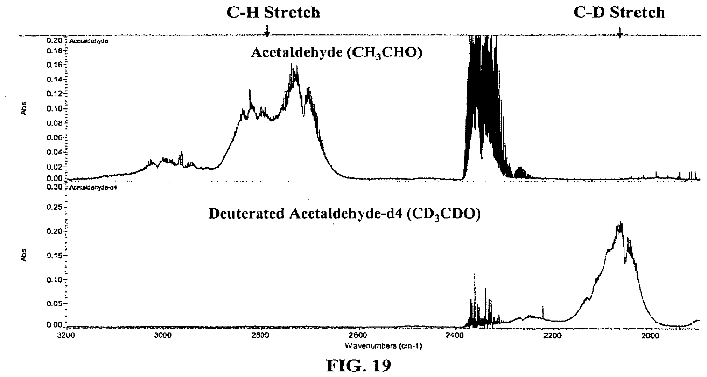

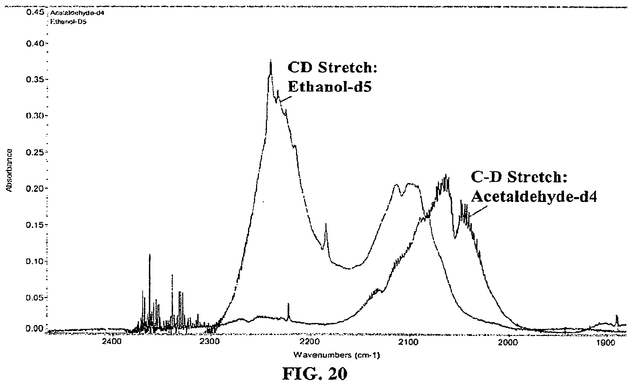

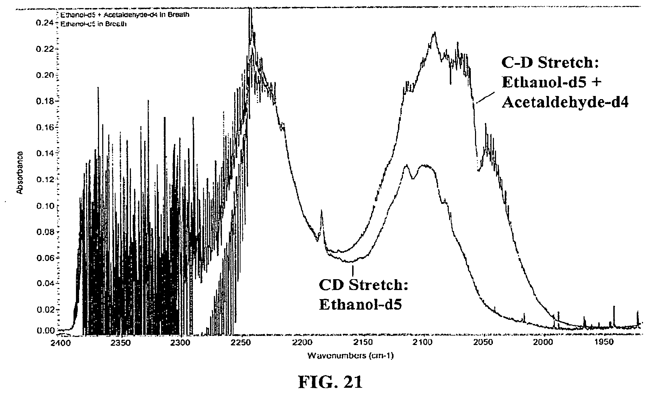

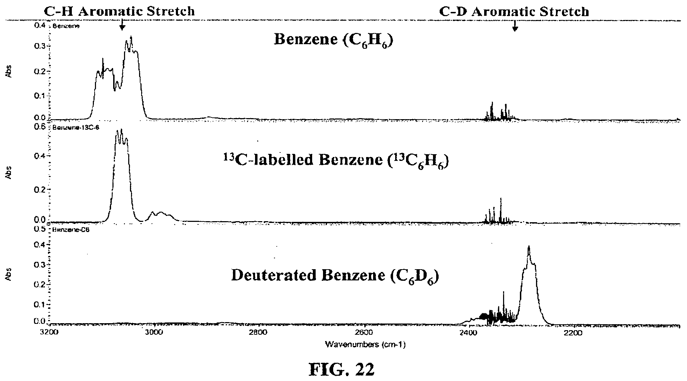

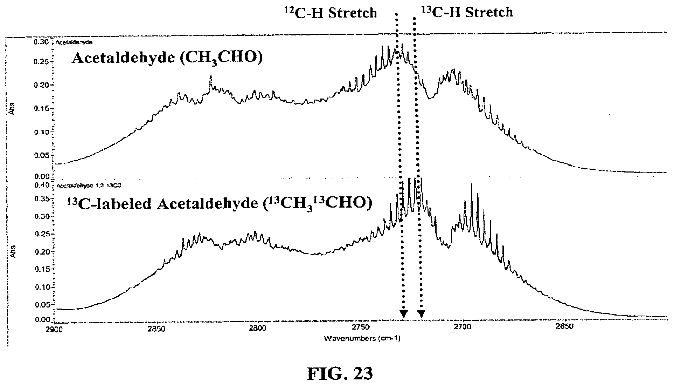

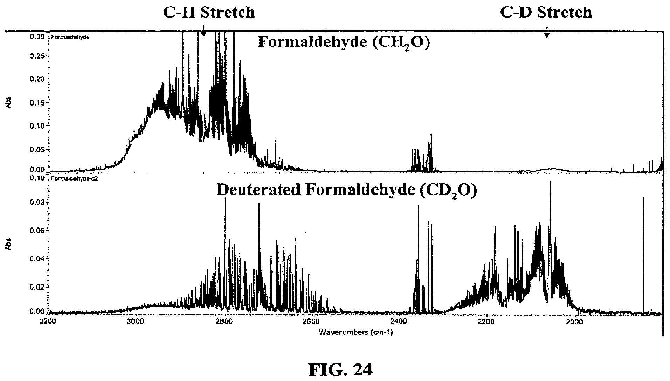

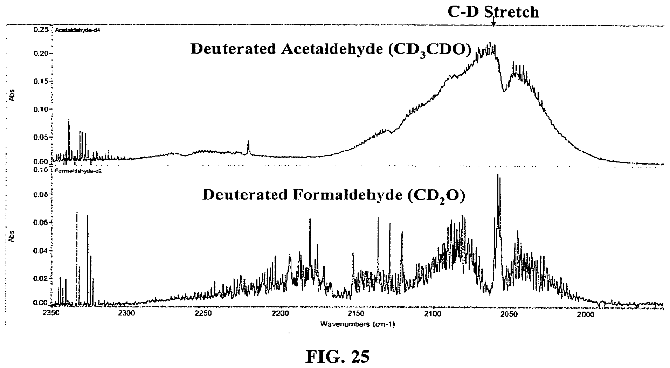

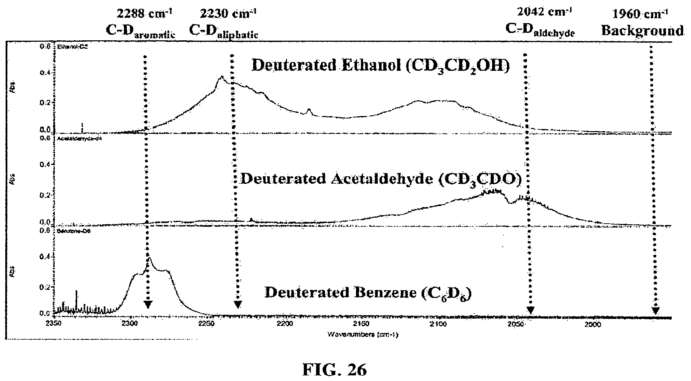

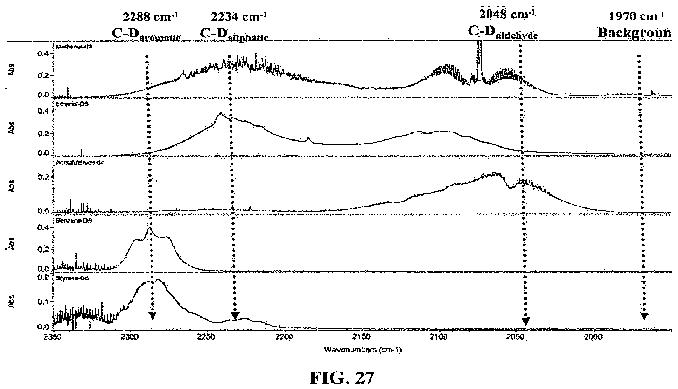

The isotopic labeling of molecular entities that serve as isotope-labeled EDIMs themselves, or of substrates that, via enzymatic degradation, liberate isotopic-labeled EDIMs, is a critically important strategy toward designing and developing an optimal MAMS. In a series of experiments (Figures 11-27) using a gas phase Fourier Transform Infrared (FTIR) device (Nicolet 6700 FT-IR, 5 liter Breath Sample, 22 meter path length) using human breath and a nitrogen environment, key scientific assumptions that underlie the advantages listed above were tested for isotopic labeling in MAMS. Specifically investigated was the effect of non-ordinary isotopic (e.g., deuterium, ¹³C; see Table 2) labeling on the FTIR spectrum of key alcohols and aldehydes on the FTIR spectrum, relative to those containing ordinary isotopes at room temperature. Important findings include: **1)** FTIR poorly discriminates between deuterated and ordinary alcohols of similar structure; the FTIR absorption spectrum for ordinary methanol and ethanol as well as deuterated methanol and ethanol are very similar. **2)** FTIR spectra for a given alcohol (ordinary vs deuterated) is highly distinctive and can be used to discriminate among them (i.e., CD₃-OH vs CH₃-OH or CD₃D₂-OH vs CH₃CH₂-OH). In contrast, GC-MS can easily distinguish between all these species. A gas chromatograph, including a miniature gas chromatograph (mGC), can easily distinguish between specific alcohols but not among deuterated and non-deuterated alcohols of a given type. **3)** FTIR does not provide a high degree of discrimination between deuterated and ordinary aldehydes of similar structure; the FTIR absorption spectrum for ordinary formaldehyde and acetaldehyde as well as deuterated formaldehyde and acetaldehyde are similar. **4)** FTIR spectra for a given aldehyde (ordinary vs deuterated) is highly distinctive and can be used to discriminate among them (e.g., CD₃CDO vs CH₃CHO or CD₂O vs CH2O). Taken together, the results indicate that isotopic labeling shows great promise in the specific, selective and sensitive detection of EDIMs in human breath for MAMS. Liquid based IR measurements of many of the same analytes discussed in Figure 11-27 displayed a similar pattern of IR spectral shifts as that determined in the gas phase, indicating measurements of these and other analytes (e.g., larger isotopic-labeled metabolic fragments of Class 1, 2 and/or 3 drugs), which may not be volatile, may be feasible with liquid-based IR measurements using exhaled breath condensate (EBC). Note: In addition, we recently carried out identical FTIR using the ketone, acetone. We found that per deuterated acetone gave a highly distinctive spectra relative to ordinary acetone (data not shown). This shows that strategies to generate ketones from 2° alcohols and/or from 2° alcohol-based esters (e.g., isopropyl-based, 2-butyl-based, 2-pentyl-based esters) show great promise as EDIM, particularly because in humans they tend to persist in the blood and breath for longer periods of time, relative to aldehydes (see below for discussion).
In summary from the above gas phase FTIR experiments ((Figures 11-27)), it appears that at least 3 fundamentally different deuterated EDIMs could be distinguished by designing a tunable midIR laser (to measure C-D vibrational stretch) with a center wavenumber of approximately 2150 ± 10% range (wavenumber range: preferably 2000 to 2300 cm-1). These EDIMs include: 1) **carbonyl** (e.g., acetone with per deuterations on methyl groups) - wave number preferably 2040 cm-1; 2) **aliphatic** (e.g., 2-butanone with deuterations on non-alpha carbons - wavenumber preferably 2240 cm-1), and 3) **aromatic** (e.g., benzaldehyde, with per deuterations on ring - wavenumber preferably 2290 cm-1. By combining molecules with molecular attributes including carbonyl, aliphatic and/or aromatic properties, at least 6 different types of molecules could be readily detected using tunable or non-tunable mIR approaches: 1) carbonyl only, 2) aliphatic only, 3) aromatic only, 4) carbonyl + aliphatic, 5) carbonyl + aromatic; 6) aliphatic + aromatic, and 7) carbonyl + aliphatic + aromatic. With the use of other types of optical detection systems, including but not limited to quantum cascade lasers, lead salt lasers, frequency-combed based systems, cavity-enhanced optical frequency comb spectroscopy, mode-locked femtosecond fiber lasers, and virtually imaged phase array (VIPA) detectors, a very large numbers of analytes, particularly in the breath, could be potentially detected.

**Element 3** - communication link and storage: HIPAA compliant information flow from the sensor to a monitoring entity to verify medication adherence and log data. This element was previously discussed in the prior patent applications listed in Introduction (Section A).

### Characteristics of an isotope-based MAMS

The preferred embodiment of an isotope-based MAMS will be designed to function under the following constraints: 1) work with all oral dosing schedules, 2) function with all orally administered drugs, 3) use a sensor having the ability to detect and measure labeled-EDIM(s) in breath samples; preferably, the sensor is a modified and optimized device such as GC or mGC with IR capabilities, IR alone, or GC-MS, and 4) be potentially coupled to existing biometric technologies (including but not limited to fingerprint, facial geometry, retinal scan, facial blood flow, or video phone) if a high degree of certainty is required. Although in the preferred embodiment, MAMS would be developed for orally ingested drugs, it could be readily applied to other modes of drug delivery (e.g., intravenous, ophthalmologic). Also, in certain preferred embodiments, the sensor is portable and provides rapid sensitive and specific measurements of labeled (preferred isotope being deuterium)-EDIM(s) breath concentrations,

### Chemistry of isotope-based MAMS

A complete isotopic-based MAMS using human breath requires that breath arising from the lungs interfaces with a sensor that measures a volatile (including semi-volatile or poorly volatile) marker, the isotope (Table 2)-labeled EDIM which is generated via different types of enzyme(s) (Table 1) following oral ingestion of a therapeutic agent and confirms adherence. The isotopically-labeled EDIM could arise from isotopic labeling of either Class 1, 2 or 3 type drugs (Section A), or any combination of them. In order to generate an isotope-labeled EDIM, the isotope could be placed on Class I, 2 or 3 drugs in the following ways: 1) a single isotopic label on a single functional group in the molecule (e.g., including but not limited to a single deuteration to replace an ordinary H atom in a methyl group), 2) multiple isotopic labels on a single functional group in the molecule (e.g., multiple deuterations to replace all of the ordinary H atoms in a methyl group), and/or 3) combinations of 1 and 2 on greater than one functional group of the molecule.

A methyl group could be used as the functional group for isotope labeling; although, any chemical group including but not limited to ethyl, propyl, and/or butyl groups on Class 1, 2, and/or 3 molecules could be used as a functional group for isotope labeling. One of the preferred embodiments would be isotopic-labeled Class 1 (GRAS type) compounds including those approved as food additives in the United States (Table 3), inactive ingredient list (Table 4), and/or excipients (Table 5). In particular, food additives used in industrialized countries, including but not limited to the compounds listed in the following tables for esters (Table 6), aliphatic higher alcohols (Table 7), aromatic alcohols (Table 8), thioalcohols (Table 9), thiols (Table 9), fatty acids (Table 10), aliphatic higher aldehydes (Table 11), aromatic aldehydes (Table 12), ethers (Table 13), thioethers (Table 14), phenol ethers (Table 15), ketones (Table 16), phenols (Table 17), lactones (Table 18), terpens (Table 19), aliphatic higher hydrocarbons (Table 20), furfurals (Table 21), indoles (Table 21), and isothiocyanate (Table 21). In addition, a number of other suitable compounds exist, including carbonate esters, acetals and ketals. Examples of these chemical classes that are food components in industrialized countries are listed in the Leffingwell & Associates (Canton, GA) "Flavor-Base 2007."

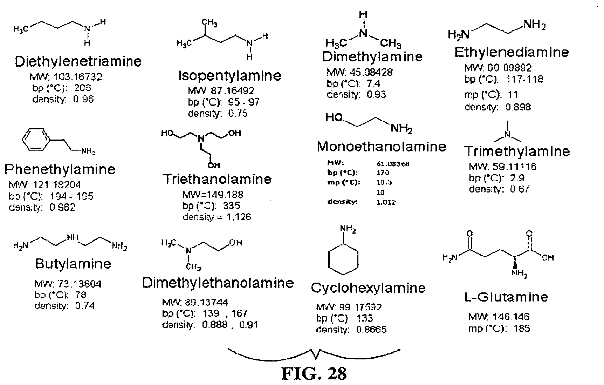

Figure 28 lists twelve amines that are listed by the FDA as chemicals found in food. A number of drugs are amines, including antihistamines (e.g., chlorpheniramine), antipsychotics (e.g., chlorpromazine), decongestants (e.g., ephedrine and phenylephrine) and central nervous stimulants (e.g., amphetamines, methamphetamine, and methcathinone). Likewise, the active therapeutic drugs contained within many FDA approved medications (Table 22) have the potential themselves to liberate suitable EDIMs for MAMS. Examples of how isotope-labeled Class 2 and 3 molecules can serve as EDIMs will be illustrated in detailed below in Section C. Last, a number of preservatives (Table 23) are added to food stuffs to maintain food quality and/or as excipients to lengthen drug life. The sources for the above compounds that are found in food, include the archives of regulatory agencies within industrialized countries such as the United States, European Union and Japan.

Not all the compounds listed in the tables would be proposed to be used for MAMS applications. Their use will be strictly governed by government regulations, toxicological information and performance characteristics with regard to MAMS function. In fact, a number of compounds, independent of taste or flavor, appear in food because they provide specific roles in food processing and/or are present as by products in the manufacturing process of creating foods. The major functions of food additives include: acidity regulator, anticaking agent, antifoaming agent, antioxidant, bulking agent, carbonating agents, clarifying agents, colloidal agents, color, color retention agent, concentrate, emulsifier, firming agent, flavor enhancer, flour treatment agent, foaming agent, freezant, gelling agent, glazing agent, humectant, liquid freezant, packing gas, preservative, propellant, raising agent, stabilizer, sweetener, and thickener.

### Development of Isotopic-Labeled EDIMs for MAMS

As described above and to summarize, the isotopic-labeled EDIM(s) can be generated by 8 different sources: the active therapeutic drug **A,** which is metabolized to a key metabolite **A1** plus other irrelevant metabolites; a salt **S,** which is potentially metabolized to a key metabolite **S1** plus other irrelevant metabolites; and drug excipient(s) **E,** which is potentially metabolized to a key metabolite **E1** plus other irrelevant metabolites; and a taggant(s) without pharmacological activity called **T,** which is located outside the matrix of the active therapeutic agent (and thus does not alter the formulation of the active therapeutic drug that was approved by the FDA) is metabolized to a key metabolite **T1** plus other irrelevant metabolites. These reactions are summarized below:

| | |
|---|---|
| ***Active Therapeutic Drug Matrix:*** | Active Drug: A → A1 + others |
| | Drug salt: S → S1 + others |
| | Drug Excipient: E → E1 + others |
| ***Taggant Compartment:*** | Taggant: T→ T1 + others |

Option 1: EDIM Source 1_{A} - Detection of A: MAMS would be designed to detect a single pharmaceutic, A. Specifically, an isotope-based MAMS using labeled A as the EDIM would be used. In certain instances, A may be present in breath too long (many hours to days) for adherence purposes, particularly with the emphasis of developing QD (oral dose once per day) or BID (oral dose twice per day) drugs, and therefore, not discriminate when individual doses were taken (due to long metabolic long half and minimal increase in plasma concentrations with dosing), which is likely given that most drugs now used are given once or twice daily.

Option 2: EDIM Source 2_{A} - Detection of A1: Option 2 (detection of an isotopic-labeled EDIM as a major metabolite of A) has multiple advantages. First, if A1 were promptly detected in breath, and were created by the action of a specific enzyme (e.g., enzyme located in the liver), it would guarantee drug ingestion since A would be absorbed into the blood and sent to the liver for metabolism. In contrast, if the subject just "chewed" the tablet in his/her mouth and tried to fool the system, the EDIM would not appear because the enzyme is not located in the saliva. Second, this type of isotope-labeled EDIM, when accurately quantitated with a breath sensor, would not only indicate medication adherence but also create a "smart" (self monitoring and reporting therapeutic) drug that could potentially report its own metabolism, and thus minimize the impact of adverse drug reactions (ADRs), secondary to drug-drug interactions (DDIs), genetic abnormalities (polymorphisms) and/or pathophysiological disturbances, in patients. Physiological factors that markedly increase or decrease the isotope-labeled EDIM concentration in breath would indicate that the metabolism of the active therapeutic agent (A) is being significantly altered and should be promptly investigated, particularly if the EDIM breath concentration was stable for a prolonged period of time before the change.

In one embodiment, the concentration of the Source 2_{A} EDIM could be used alone to follow the *in vivo* metabolism of A. In a second embodiment, to rule out or to minimize factors such as alterations in gastric emptying (e.g., fatty meal, stress, etc.) and/or variable absorption causing changes in Source 2_{A} EDIM breath concentrations and causing false alarms that the active therapeutic drug (A) is not being metabolized properly, a comparator compound could be included in the pill matrix, which is metabolized by a different enzyme (location, capacity, and/or function), and would generate another EDIM independent of that from the active therapeutic drug. For example, lets assume isotope-labeled A is metabolized by the most important CYP450 enzyme (lower capacity) for drug metabolism, CYP-3A4 to isotope-labeled A1 (Source 2_{A} EDIM) whereas the comparator is metabolized by the high capacity enzyme butyrylcholinesterase to another EDIM. If the ratio of the maximum concentration of the EDIM from the active therapeutic drug to that of the comparator were constant, it is likely the active drug is being metabolized properly. In other words, if the breath isotope-labeled A1 EDIM concentration was reduced, but there is a parallel decrease in the comparator's EDIM, it would indicate a physiological change such as delayed gastric emptying, which certainly is unrelated to drug metabolism. In contrast, if the ratio markedly changes, it would indicate a potential problem: ratio (A1/comparator) increases - enhanced metabolism of A; versus ratio (A1/comparator) decreases - reduced metabolism of A). Furthermore, like the case in Option 1, the metabolites of the active pharmaceutic, A1 would have the same disadvantages as outlined above.

In another embodiment, by adding different EDIM or combinations of EDIM in addition to those mentioned above, we can conceive of a "genius" level New Intelligent Chemical Entity (NICE) molecule that not only reports its ingestion but also the dose and its metabolism on an ongoing basis. The FDA is highly supportive of improving drug safety. For example, in a recent publication the Center for Drug Evaluation and Research (CDER) stated that the central issue is not whether pharmacogenomic-guided drug prescriptions will happen (Lesko-LJ and Woodcock-J, Pharmacogenomic-guided drug development: regulatory perspective, The Pharmacogenomics Journal (2002) 2, 20-24), but when and where. By ensuring drug adherence and monitoring metabolism with NICE-type drugs, the MAMS technology outlined in this invention will take drug safety to another level.

Moreover, this type of "smart" medication would be naturally the most intelligent at reporting its metabolism, relative to pharmacogenomic-based approaches and/or using enzyme metaprobes (e.g., phenotypic, breath-based tests: ¹⁴C-erythromycin for CYP-3A4, or ¹³C-caffeine for CYP-1A2) to elucidate its ability to be metabolized by a given enzyme. Why does this occur? First, blood tests to examine for genetic-based defects in enzyme function (e.g., particular CYP fractions such as 2D6, 2C19 having genetic polymorphisms) only cause problems in a subset of the CYP isoforms and doesn't involve the most important CYP enzyme, CYP3A4. Second, genetic-based tests ignore an even more important cause of ADRs - drug-drug interactions (DDIs). A patient with a normal genome for a specific enzyme (e.g., normal AmpliChip result being proposed to individualize drug therapy) could be placed on a therapeutic agent that is metabolized by this same enzyme, even though his/her enzyme activity may be actually less than 5% of normal activity due to a DDI! As a result, he/she may suffer drug-related morbidity and mortality due to a DDI. In a manner being proposed for genetic tests, subjects can be stratified into the following metabolic categories using EDIMs generated from NICE-type therapeutic agents: 1) poor metabolizers, 2) intermediate metabolizers, 3) extensive metabolizers, and 4) ultrarapid metabolizers. Third, many drugs are degraded by multiple enzymatic pathways, including multiple CYP fractions or combinations of CYP and non-CYP enzymes. In many cases it is overly simplistic to focus on the activity of only one enzyme. Thus, genetic tests and breath tests that examine the function of a specific enzyme may not provide an accurate assessment of the net effect (or integrated action) of various degradation pathways and by consequence drug pharmacokinetics and levels. Because many drugs have more than one metabolic route, the EDIMs of smart drugs mentioned in the current invention will not necessarily measure the metabolic rate of any one particular physiological process, but rather identifies and integrates all relevant metabolic pathways in a manner that identifies whether a drug is being properly metabolized in an on-going continuous manner.

If one examines the CYP450 enzyme system, all of the FDA-approved drugs listed in Figures 7-10 have the potential to be converted into "smart" self-reporting (NICE-type) therapeutic molecules. Nevertheless, EDIM Source 2_{A} still limits the system to detecting ingestion of a specific active drug (i.e., one EDIM approach doesn't fill all MAMS needs). Likewise, because so many FDA approved drugs produce common metabolites, particularly formaldehyde via desmethylation reactions (Figure 7), the EDIMs would be similar and may not be able to distinguish among the different therapeutic agents shown in Figure 7. Furthermore, like the case in Option 1, the metabolites of the active pharmaceutic, A1 would have the same disadvantages as outlined above.

In a preferred embodiment, a "genius" level NICE molecule is provided that not only reports its ingestion but also the dose and its metabolism on an ongoing basis. Note: In this application, the use of stable isotopic labeling (e.g., deuterium) to generate EDIMs, should have minimal-to-no impact on chemistry-manufacturing controls (CMC) or active pharmaceutical ingredients (API), and thus should invoke minimal regulatory scrutiny.

In another embodiment, the technology described in the current application could be used to provide a new and novel basis, independent of existing technologies (e.g., scintigraphic tests that scan the stomach with radiographic equipment, or breath-based ¹³C-octanoate tests that measure expired ¹³CO₂ with expensive analytical devices), of non-invasively measuring gastric emptying using ordinary and non-ordinary isotopic-labeled EDIM(s) and a sensor to measure them. The test would simply require a subject to exhale breath into a sensor for a short period of time at intermittent times, immediately before and after ingesting a pill. For example, consider a pill system, which contains 3 key chemical design elements, that will interface with a sensor to measure the concentration of EDIMs in the breath: 1) "mouth" chemicals to "time stamp" entry of the pill system into the mouth, 2) "stomach" chemicals to "time stamp" entry of components of the pill system into the stomach, and 3) "small intestine" chemicals to "time stamp" entry of components of the pill system into the small intestine.

**Mouth Chemicals:** Mouth chemicals surface coated on (one preferred embodiment) or located within the pill (e.g., including but not limited to a gelatin capsule) will immediately generate a mouth-derived EDIM(s), either derived directly from the mouth chemical(s) (preferred embodiment), metabolites of the mouth chemicals, or both, upon entry into the mouth and will thus "time stamp" when the pill was placed into the mouth.

**Stomach Chemicals:** Stomach chemicals surface coated or contained within (one preferred embodiment) the pill (e.g., including but not limited to a gelatin capsule) will be quickly released from the capsule (preferred embodiment) and be significantly absorbed into the blood via transport through the gastric wall (e.g., includes but not limited to ethanol) and will be immediately detected as a stomach EDIM and/or stomach EDIMs, either by measuring the stomach chemical(s) themselves, metabolite(s) of the stomach chemical(s) via enzyme action (preferred enzyme is located in blood and high capacity to rapidly generate the stomach EDIMs), or combinations of both. Thus, the appearance of stomach chemical-derived EDIMs will "time stamp" entry of the pill system into the stomach.

**Small Intestine Chemicals:** Small intestine chemicals coated on or contained within (preferred embodiment) the pill (e.g., including but not limited to a gelatin capsule) is a chemical or more than one chemical, hereafter called the "small intestine" chemical, that is absorbed into the blood via transport through the wall of the small intestine, preferably through the duodenum, but not through the wall of the stomach; shortly after entering the small intestine and entering the blood stream, the small intestine chemical(s) will be immediately detected as a small intestine EDIM and/or small intestine EDIMs, either by measuring the small intestine chemical(s) themselves, metabolite(s) of the small intestine chemicals via enzyme action (preferred enzyme is located in blood and high capacity to rapidly generate the small intestine EDIMs), or combinations of both. Thus, the appearance of small intestine chemical-derived EDIMs will "time stamp" entry of the pill system into the small intestine. The use of the mouth, stomach and small intestine "time stamps" described above, unlike current systems used to measure gastric emptying, will allow not only gastric emptying times to be measured non-invasively, but also allow simultaneous assessment of esophageal transit times and subsequent correction of gastric emptying times (subtracting off esophageal transit time). A number of medical conditions and/or drugs can affect esophageal transit and gastric emptying independent of one another. In addition, the described system could be further expanded to assess emptying times at other elements of the gastrointestinal tract, such as the colon where bacteria can be used to liberate unique colon EDIMs. In all of these applications, the pill system can be administered under various conditions, including fasting, standard liquid meal and/or standard fatty meals.

Option 3: EDIM Source 1_{S} - Detection of S: The major limitation of Option 3 is that MAMS would be designed to detect a single salt, S as the EDIM that is chemically part of the active therapeutic agent, A. This approach may or may not suitable for MAMS. A second disadvantage is that the physicochemical characteristics, pharmacokinetics or effective therapeutic concentrations of a salt may not be suitable for detection in the breath. One illustrative example for Option 3 would be isotopic (e.g., ¹³C, ¹⁷O, ¹⁸O and/or deuterium)-labeled acetate (acetic acid), which is frequently used as a pharmaceutical salt, and is quite volatile and may serve as a Source 1_{S} EDIM.

Option 4: EDIM Source 2_{S} - Detection of S1: Option 4 (detection of the major metabolites of S, S1 in the breath post oral ingestion) may also be feasible in some embodiments. For example, acetate (acetic acid) was mentioned in Option 3. Isotope (e.g., ¹³C, ¹⁷O, ¹⁸O and/or deuterium from Table 2)-labeled acetic acid (Figure 6) is converted to CO₂ and H₂O via the tricarboxylic acid (TCA) cycle. These metabolic products, containing isotopic-labels, could serve as a Source 2s EDIM.

As shown in Figure 7, many therapeutic agents directly generate formaldehyde via desmethylation reactions. In terms of assessing metabolic function, such as CYP activity, the production of formaldehyde, rather than CO₂, is a more accurate measure of metabolic function. By using CO₂ as the breath marker to assess enzyme competence, two additional enzyme systems (Figure 6) are brought into the picture (i.e., alcohol dehydrogenase, formaldehyde [aldehyde] dehydrogenase). If a defect in either enzyme system was present, it is possible to falsely conclude that CYP function was abnormal, versus a defect in the enzyme(s) system distal to formaldehyde. As confirmation of this problem, because the production of carbon dioxide from the oxidation of many CYP-substrates such as erythromycin is folate-dependent (Figure 6), a reduction in the folate-dependent intermediate step caused an abnormal test in the erythromycin breath test, when in reality no metabolic abnormality was found in CYP-3A4 function. The technology outlined in this application, particularly in reference to the NICE concept, may be able to exploit the use of isotopic-labeled formaldehyde (or any other aldehyde, etc) in order to directly measure CYP activity and thus avoid this potential pitfall, and provide a basis for drugs that self report their metabolism.

Option 5: EDIM Source 1_{E} - Detection of E: Many excipients (Table 5) exist that are used to optimize formulation of a therapeutic agent. Some of these agents may be suitable to provide isotopic-label Source 1_{E} EDIMs for MAMS.

Option 6: EDIM Source 2_{E} - Detection of E1: Isotopic-labeled EDIMs arising from excipients also may be used for MAMS. For example, aspartame or neotame are an ester-based artificial sweetener that liberates L-phenylalanine, aspartic acid and methanol when it is hydrolyzed.

Option 7: EDIM Source 3 - Detection of T: The presence of T may not be necessary if the EDIM can be generated by other sources. The major advantage of Option 7 (detection of T which is ingested along with a capsule containing A) is that it not only allows the selection of a chemical taggant that possesses the attributes of the ideal EDIM (see Section B.4 for details), but also it can be utilized to verify oral ingestion of *any* active pharmaceutic. However, by utilizing T, rather than a metabolite of T, T1 (Option 8), we cannot guarantee that the tablet was ingested. This drawback can be significantly mitigated or even eliminated by pill design factors.

Option 8: EDIM Source 4 - Detection of T1: In this approach for isotope-based MAMS (detection of isotope-labeled T1 as the EDIM), A and T co-exist in the same pill/capsule but in the preferred embodiment the presence of T does not alter the CMC/API of the active pharmaceutical ingredient. It has 3 major advantages: 1) allows the selection of a chemical taggant that possesses the attributes of the ideal EDIM (see Section B.4. for details), 2) can be utilized to verify oral ingestion of *any* active pharmaceutic, and 3) can guarantee that the active pharmaceutic was ingested, entered the blood, traveled to its biological target sites and via its mechanism(s) underlying efficacy exerted its therapeutic action. For example, if an enzyme which is located in the liver converts T to T1, then detection of T1 in the breath definitively confirms pill/capsule ingestion of active drug in the person who actually put the tablet in their mouth.

### Preferred Isotopic-labeled EDIM for MAMS

Independent of the source of the EDIM (see four general sources of EDIMs above), at least 12 factors should be considered when designing a system to generate the ideal EDIM for an effective MAMS:
1. Applicable to all oral administration regimens. Although the oral route is the preferred embodiment, other routes of administration may include but are not limited to non-oral routes such as intravenous, transdermal, rectal, nasal, cerebrospinal fluid, subcutaneous, intramuscular).
2. Reproducible, rapid appearance of the EDIM in breath after ingestion and absorption of the pill.
3. Reproducible duration of EDIM appearance in the breath for QD or BID dosing: duration is not greater than 5 hrs or less than 15 min.
4. The EDIM is unique in the breath (e.g., not found in multiple foods, not found normally during endogenous metabolism, and not produced in high concentration during disease) and provides a good signal to noise ratio with the detector.
5. Type of metabolism not critical but if T is used to generate the EDIM, it is preferably non-CYP (e.g., esterase) to avoid potential drug-drug interactions (DDIs); a "smart" drug has the potential to not only generate an EDIM to confirm medication adherence but also self reports it metabolism.
6. EDIM is relatively volatile to readily appear in the breath but not extremely evanescent in the blood; does not undergo very fast subsequent metabolism to nonvolatile metabolites. Ideally, the EDIM should not have a pKa value that renders the majority of the molecule to the charged state at physiological pH (pH = 7.4), which may poorly appear in the breath.
7. Has no intrinsic toxicity, pharmacological activity, or inhibitory effect on the metabolism of other compounds at concentrations required for detection. For example, subjects on disulfiram, which inhibits aldehyde dehydrogenase and causes acetaldehyde to accumulate (development of "flu-like symptoms" in humans is a negative incentive to consume alcohol), is used to treat alcoholism and may cause an aldehyde-based EDIMs to produce side effects due to higher than expected levels of the aldehyde). This scenario is very unlikely given the low dose of taggants used to generate the EDIM in MAMS, and the concentrations of EDIM formed for MAMS applications.
8. EDIMs may be of any or combination of the 3 drug classes described in Section A.
9. Ideal EDIMs should be stable in the body, be exclusively eliminated by exhalation, and not undergo additional metabolism. This criterion will almost never be met with GRAS type taggants.
10. If a taggant is used to generate the EDIM, it should not alter the PK/PD of the therapeutic agent (e.g., API has bioequivalence; API has the same biological interaction with the body, and ADME).
11. The chemical source of the EDIM should be inexpensive, readily available, and easy to synthesize.
12. The formation of the EDIM should not be easily blocked by other chemicals (e.g., therapeutic agents or non-therapeutic chemicals that inhibit the enzyme that liberates the EDIM) or pathophysiological conditions frequently present in humans.

Additional selection criteria for taggants in MAMS, which may be used to generate the EDIM, include: 1) state of matter: solid versus liquid; 2) taste: absent or present (pleasant vs unpleasant); 3) physicochemical properties: boiling point, melting point, Henry's Law constant (K_{H}); 4) PK properties: ADME, including metabolism rates and routes (non-CYP-450 to avoid adverse drug reactions [ADRs]); 5) extensive safety data: stability, toxicological data such as permissible daily exposure (PDE) in humans and LD₅₀ values in various species (typically in the gms/kg range for oral administration); 6) minimal-to-no implications from a regulatory perspective (no impact on CMC of API [study drug or FDA approved drug] or PK/PD of API); and 7) metabolism of taggant generates EDIMs that are easily detected by the appropriate detection technologies (e.g., IR) (e..g., EDIM is detected by the sensor and is neither a significant endogenous chemical nor widely generated via ingestion of different foods).

### Enzyme Chemistry, EDIMs and MAMS

To generate isotopic-labeled EDIMs via catalysis that are suitable for MAMS, a great diversity of Class 1, 2, and/or 3 drugs (described above) exist that can be acted upon by several types of enzymes. A carboxylate ester, when acted upon by an esterase(s), liberates an acid and alcohol (Figure 1). In the case of many GRAS ester taggants, the metabolites, namely an alcohol (Figure 2) and a carboxylic acid (Figure 3) can be selected as EDIMs because one or both can be volatile (or semivolatile) and thus serve as EDIMs. In addition, carbonate esters, acetals and ketals can also be used to easily generate a wide variety of corresponding alcohols and carboxylic acids as EDIMs. The 1° and 2° alcohols, generated from these compounds, will in turn, generate aldehydes and ketones, respectively. The aldehydes, and particularly the ketones may also be suitable EDIMs.
Compared to carboxylic acids, alcohols are a more suitable EDIM for a variety of reasons (e.g., carboxylic acids have poor [high] K_{H} values (=CL/CG, liquid to gas phase concentration ratio that cause them to partition preferably in blood versus breath). In the case of 1° alcohol-based aliphatic esters (1° esters) such as ethyl butyrate, esterases rapidly create a 1° alcohol (i.e., ethanol). For 2o alcohol-based aliphatic esters (2o esters) such as 2-pentyl butyrate, they are rapidly hydrolyzed to their corresponding 2o alcohol (i.e., 2-pentanol) by esterases, particularly by carboxylesterases (e.g., β-esterase). The carbon that carries the hydroxyl (-OH) group of primary (1°), secondary (2°) and tertiary (3°) alcohols is attached to 1, 2, and 3 alkyl groups, respectively. The 1° and 2° alcohols are primarily converted (oxidized) via alcohol dehydrogenase (ADH) to their corresponding aldehydes and ketones, respectively. In contrast to 1° and 2o alcohols, 3o alcohols, due to steric hindrance with ADH, are very resistant to metabolism in humans and thus are not ideal for MAMS, unless a 3o alcohol-based ester liberated a 3o alcohol (e.g., tert-butyl butyrate to tert-butanol), which was used as the EDIM. The aldehydes are further metabolized by aldehyde dehydrogenase (ALDH), which oxidizes (dehydrogenates) them to their corresponding carboxylic acid. In contrast, methyl ketones undergo α-hydroxylation (e.g., conversion of 2-butanone [methyl ethyl ketone, MEK] to 3-hydroxy-2-butanone [acetoin] via CYP-2E1 and CYP-2B, or conversion of 2-pentanone [methyl propyl ketone, MPK] to 3-hydroxy-2-pentanone) and subsequent oxidation of the terminal methyl group to eventually yield corresponding ketocarboxylic acids. Unlike aldehydes, disulfiram (an inhibitor of ALDH) should not inhibit the metabolism of ketones. The ketoacids are intermediary metabolites (e.g., α-ketoacids) that undergo oxidative decarboxylation to yield CO2 and simple aliphatic carboxylic acids.

The acids may be completely metabolized in the fatty acid pathway and citric acid cycle.

It appears that 2° alcohols (or even 2° esters that generate 2° alcohols) are excellent taggants for definitive adherence monitoring, and appear superior to the simple 1° alcohols in this respect. The presence (and persistence) of their corresponding ketones (EDIMs) in exhaled breath represents definitive proof of ingestion of a medication containing 2° alcohols (or a 2° alcohol-based ester, carbonate ester, ketal, etc.) as taggants. In general due to increased steric hindrance, 2° alcohols are not as good substrates for ADH relative to 1° alcohols. Likewise, the enzymatic pathways to degrade alcohol-derived ketones appear less efficient than those for alcohol-derived aldehydes. Given the fact that 1) the gastric wall has a high concentration of ADH and alcohols (e.g., ethanol) are known to be significantly absorbed through the stomach, and 2) alcohols undergo extensive first pass metabolism via ADH in the liver after absorption from the GI tract, it should not be surprising that 2-butanone levels appear very rapidly in the breath, and its concentrations significantly exceeds those of 2-butanol (ketone:alcohol ratio: 2-butanone/2-butanol >>1).

In addition, other 2° alcohols will increase the number of taggants available for definitive adherence monitoring. In keeping with our hypothesis that 2° alcohols (vis-à-vis 1° alcohols) would generate ketones that would persist in the body and have significant excretion by the lung, diabetic patients readily excrete ketones during the pathophysiological condition of diabetic ketoacidosis (DKA). Therefore, it should not be surprising that ketones generated from other sources (e.g., orally ingested 2° alcohols) would also be excreted by the lung. Using mGC-MOS we have already shown these endogenous DKA-related ketones are easily distinguished from the ketones, which would be generated from 2° esters or alcohols, including 2-butanone and 2-pentanone (data not shown).

Below is a summary of some key advantages and disadvantages of using esters, 1° alcohols and 2° alcohols for definitive MAMS:

### A. Esters

### Advantages

- Great variety of GRAS food additives
- Esterases generate corresponding alcohol and carboxylic acid via enzyme systems that are widely present in humans and not easily saturable
- Many exist in liquid and solid state forms
- Relative to alcohols, many more choices for selecting solids
- Great variety of favorable tastes
- 2° alcohol-based esters such as 2-pentyl butyrate are primarily degraded by carboxylesterase to 2-pentanol and butyric acid

### Disadvantages

- Greater mass of taggant required to be interfaced to API in order to generate a fixed mass of EDIM (e.g., 2-butanone)
- Some esters not optimal from a stability standpoint
- 1° alcohol-based esters as GRAS compounds are much more common than 2° alcohol-based esters in food databases; these alcohols generate aldehydes, which are not ideal EDIMs relative to ketones derived from 2° alcohols

### B. 1° alcohols

### Advantages

- Much greater variety of GRAS food 1° alcohols relative to 2o alcohols
- Larger 1° alcohols via ADH generate aldehydes, particularly those that are branched, which are better EDIMs (e.g., low KH values; distinct from endogenous compounds) than more simple 1° alcohols, but have lower vapor pressures

### Disadvantages

- ADH forms aldehydes from 1° alcohols, which are generally not as good EDIMs as ketones, particularly with the more simple 1o alcohols
- Many have classic alcohol taste; may require CMC architecture approaches or addition of taste "maskers" to avoid
- Disulfiram, a drug used to treat alcoholism that blocks the action of aldehyde dehydrogenase, may interfere with the degradation of corresponding aldehydes, and cause side effects; this effect is expected to be clinically irrelevant due to the small mass of alcohol (or its corresponding ester) required for definitive MAMS
- Ethanol consumption (via interaction with ADH) can theoretically reduce the conversion of 1° alcohol taggant to its corresponding aldehyde; this has not been found to be clinically significant for a number of non-ethanol alcohols (excludes methanol)

Note: In addition of 1o alcohols, a number of critically important CYP-450 metabolic reactions for pharmaceutical agents, via dealkylations, generate various aldehydes, include formaldehyde via desmethylation, acetaldehyde via desethylation, propionaldehyde via despropylation, and butyraldehyde via desbutylation.

### C. 2° alcohols

### Advantages

- ADH generates ketones, which generally have more favorable physicochemical and metabolism characteristics as EDIMs than aldehyde ones
- Disulfiram, an inhibitor of aldehyde dehydrogenase, will not interfere with the degradation of ketones formed from 2o alcohols (e.g., methyl ethyl ketone, derived from 2-butanol, is converted to 3-hydroxy-2-butanone via CYP-2E1 and 2B).

### Disadvantages

- Many have classic alcohol (ethanol) taste; may require CMC architecture approaches or addition of taste "maskers" to avoid
- Few 2° alcohols, relative to 1o alcohols, are listed in GRAS food databases
- Few 2° alcohol-based esters are listed in GRAS food databases (e.g., these would generate the 2° alcohol, and later a ketone)

Because esterases (unlike the CYP450 system) are high capacity enzyme systems, their exploitation for MAMS is desirable, as the presence of ester taggant(s), if used and necessary, is not likely to cause drug-drug interactions (DDIs) or have its function in MAMs be adversely impacted by DDIs, when co-administered with therapeutic agents.

The vast majority of pharmaceutics are metabolized by CYP450 (Figure 4 lists some key P450 reactions), particularly CYP-3A4 and CYP-2D6. Likewise, the redundancy of various esterase functions in humans (a drug can be metabolized by different esterases) is desirable to avoid the impact of genetic alterations in enzyme function (e.g., genetic polymorphisms with CYP-2D6, CYP-2C9 and/or CYP-2C19) that could adversely impact a MAMS system using CYP-based taggant substrates. On the other hand, in the case of NICE-type drugs that are metabolized by the CYP450 system, when the active therapeutic "self reports" not only its ingestion but also its metabolism, it is of course desirable and necessary in this situation for the EDIM to be derived from the CYP450 enzyme system, in order for the drug to be "smart".

The breath kinetics (presence, rapidity of appearance, concentration, duration of presence, etc) of the isotope-labeled EDIM(s) is a function of the following factors: 1) dose of Class 1, 2 or 3 drug(s) used as the EDIM(s) or generating the EDIM(s) (via enzymatic action), 2) the rate of liberation of the EDIM(s) via enzyme action into the blood, 3) rate of removal of the EDIM via non-breath endogenous metabolic routes (e.g., conversation of alcohols, aldehydes and carboxylic acids generated by enzymes such as esterases or CYP450 to CO₂ and H₂O via the TCA cycle; see Figure 2) and 4) intrinsic EDIM properties (*e.g.,* physicochemical properties such as vapor pressure and pharmacokinetic features such as metabolic half life, clearance, volume of distribution, pKa).

Many of the EDIMs, which have suitable physicochemical properties (e.g., volatility, duration of appearance in breath, etc.) for MAMS, are already present in the blood and breath of humans as part of endogenous metabolism and/or diet. This applies to a variety of chemically diverse substances, including but not limited to alcohols, fatty acids, aldehydes, ketones. For example, in humans the endogenous blood and breath concentrations of ethanol, methanol and formaldehyde are shown below:
***Ethanol*** - Blood plasma: 4000-33000 nM (Jones-AW, J Anal Toxicol, 9:246, 1985) versus Breath: 2.2 to 6.5 nM (Phillips-M and Greenberg-J, Anal Biochem, 163:165-169, 1987).
***Methanol*** -: Blood plasma: 12500-25000 nM (Jones-AW et al, Pharmacol Tox 66:62-65, 1990) versus Breath: 4.6-9.2 nM (Jones-AW et al, Pharmacol Tox 66:62-65, 1990)
***Formaldehyde*** - Blood plasma: 13000-20000 nM (Szarvas-T, J Radioanalytical and Nuclear Chem, 106:357-367, 1986) versus Breath: 12-24 nM (Ebeler-SE, J Chromatogr B Biomed Sci Appl, 702:211-215, 1997).

Methanol is thought to be formed from the microflora of the gastrointestinal tract and from dietary intake. Methanol may not be suitable for MAMS due to the effects of ethanol on completely or nearly completely blocking methanol oxidation. Ethanol is the preferred substrate of alcohol dehydrogenase and its presence in the blood after imbibing ethanol will markedly lengthen the half life of methanol in the body. For example, methanol has a half life in the blood in the absence of alcohol of 1.8-3.0 hrs versus greater than 8-24 hrs in the presence of alcohol (Haffner et al, Int JLegal Med, 105:111-114, 1992), making it potentially unsuitable as an EDIM. The half life of the EDIM has to be short enough so its presence does not interfere with subsequent dosing, which is obviously more of a problem with drugs given more than once per day. A number of other volatiles resulting from endogenous metabolism can also be found in human breath. For example in one study (Diskin-AM et al, Physiol Meas 24:107-119, 2003), the mean concentrations, in parts per billion (ppb), for the listed compounds were: ammonia, 422-2389; acetone, 293-870; isoprene, 55-121; ethanol, 27-153; acetaldehyde, 2-5. If the above molecules contained only ordinary isotopes and were proposed as EDIMs, it may be difficult in many cases to distinguish them from background levels (poor signal to noise ratio), and thus they would not be ideal for MAMS application. In contrast, if we used non-ordinary isotope-labeled EDIMs, these entities can be readily distinguished from the endogenous compounds on the basis of various techniques, including but not limited to infrared spectroscopy (see Figures 11-27) or mass spectroscopy.

Examples of appropriate infrared spectrometers include but are not limited to those disclosed in U.S. Patent 5,063,275, herein incorporated by reference. For example, to illustrate this concept, if deuterated (or carbon labeled) formaldehyde could be readily discriminated from endogenous (background) formaldehyde and if enough labeled formaldehyde, when liberated via CYP-mediation oxidation of the parent compound, can escape the metabolic machinery of cells (conversion of formaldehyde to formic acid and CO₂, Figure 6) and flow into the blood, this would allow medication adherence and even the metabolism to be monitored of many, if not all, of the FDA-approved drugs listed in Figure 7. On the other hand, if this turns out not to be the case and given the fact that the CYP system is easily saturable and frequently causes ADRs (and morbidity and mortality) due to DDIs and genetic abnormalities, the current invention may provide an impetus for pharmaceutical companies to create new chemical entities that are degraded by non-CYP450 (preferred embodiment) but also by CYP450 pathways to generated EDIMs, which allow for the molecule to be "smart" (i.e., it would self report adherence and metabolism).

Although the Katzman technology (United States Patent 5962335) teaches how to predict using isotope breath tests whether a drug will be properly metabolized by a subject prior to being placed on that particular drug (proposed individualized therapy), the invention described herein differs in many important respects. First, Katzman does not teach how to use isotopic labeling for purposes of assessing medication adherence, including frequency of drug dosing and dosage of a medication. Second, we propose to invent a new class of therapeutic agent, termed "smart drugs" or NICE-type agents, which continuously monitors not only medication adherence, but also its ability to metabolize themselves in a continuous manner. Thus, in the preferred embodiment we propose continuous monitoring and not a single *a prior* assessment of metabolic competence before being placed on the medication, which is the approach taken by Katzman. It is likely that most ADRs, such as DDIs or physiological abnormalities would occur while on drug therapy, particularly for those drugs which are taken over a patient's lifetime. Third, the actual physical material of active therapeutic agent that is being used to assess metabolic competence is actually being used to treat the patient's medical disorder. In other words, our invention will preferably use the active therapeutic drug itself when it is being actually used to treat disease to simultaneously monitor its own metabolism, as opposed to the Katzman approach of using an exogenously administered test drug probe (the drug here is not being used to treat medical disorders) to assess metabolic competence. The principal of NICE type agents, where a fragment of the parent FDA approved drug, can be used to assess medication adherence and metabolism, while it is simultaneously treats disease, is completely novel. Fourth, Katzman proposes to use the most distal products of metabolism, such as labeled CO₂ and NH₃, but does not explicitly mention the use of more proximal ones, including but not limited to alcohols, acids, aldehydes, and/or ketones, which is a preferred embodiment of the current invention. Fifth, Katzman does not explicitly mention the use of deuterium, which is a preferred embodiment as an isotopic label for EDIMs. In summary, unlike the current invention, it was not intent of Katzman to create a new class of "smart" therapeutics agents, based on selective isotopic (preferably non-radioactive, Table 2) labeling that does not alter the active drug's PK/ADME or pharmacodynamics, which is given chronically (or acutely) to treat disease while simultaneously and continuously monitoring adherence and metabolism.

### Illustrative Examples of How Isotope-labeled EDIMs Can Be Enzymatically Generated from Class 1, 2, and 3 Drugs

As outlined above, a number of strategies can be used to create a variety of flexible medication adherence systems (MAMS), some of which may involve chemistry that will allow the therapeutic molecules to be "smart" since they will treat disease while simultaneously monitoring adherence and metabolism. In this section, we describe 3 enzyme systems, namely esterases, CYP450 and deaminases, which can generate isotopic-labeled EDIMs. Each enzyme will be following by selected illustrated examples of how Class 1, 2 and 3 drugs can be used in MAMS. Note: In most cases, the parent molecule will be broke down in a smaller more volatile fragment(s), which is the preferred embodiment for the EDIM(s), but does not exclude using the larger metabolic fragment(s) as an EDIM(s).

### Esterases

Esters (EC 3.1.1) are hydrolyzed without the requirement of molecular oxygen by esterases to a carboxylic acid and an alcohol (Figure 1). Esterases, hydrolases which split ester bonds, hydrolyze a number of compounds used as drugs in humans. The enzymes involved are classified broadly as cholinesterases (including acetylcholinesterase), carboxylesterases, and arylesterases, but apart from acetylcholinesterase, their biological function is unknown. The acetylcholinesterase present in nerve endings involved in neurotransmission is inhibited by anticholinesterase drugs, e.g. neostigmine, and by organophosphorous compounds (mainly insecticides and chemical warfare agents). Cholinesterases are primarily involved in drug hydrolysis in the plasma, arylesterases in the plasma and red blood cells, and carboxylesterases in the liver, gut and other tissues. By incorporating various isotopic labels listed in Table 2 (preferred embodiment is deuterium), where appropriate, into the various atomic sites of the esters, various EDIMs (arising from the ester, acid and/or alcohol, and their corresponding ketone/aldehyde) containing one or more isotopic labels could be generated that will fulfill the requirements of an effective MAMS.

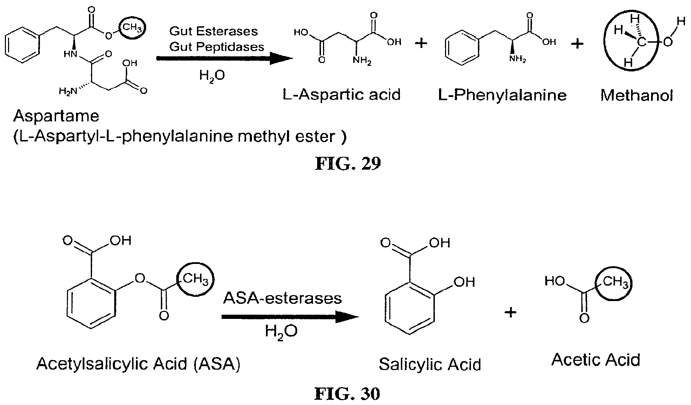

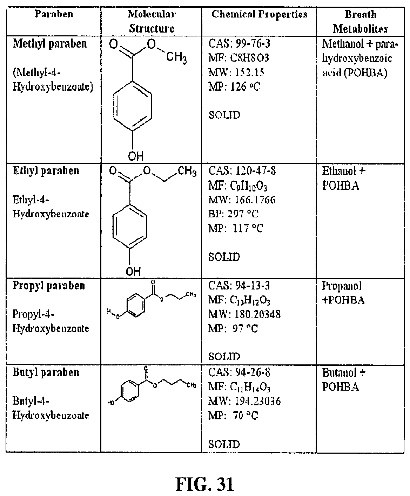

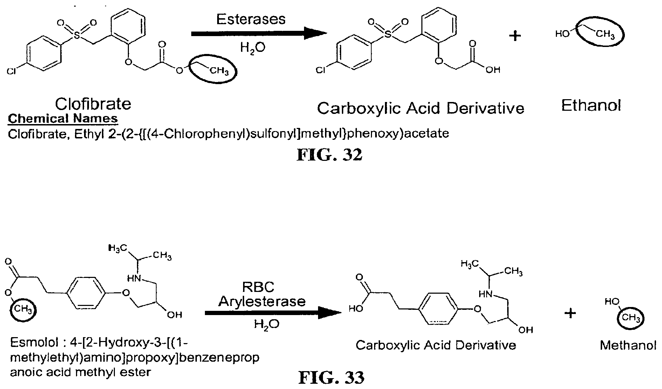

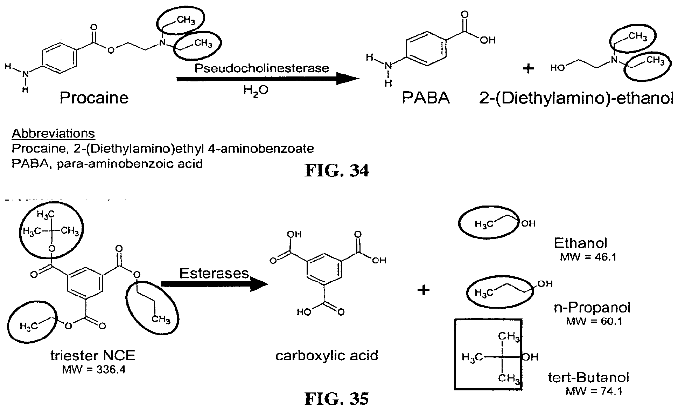

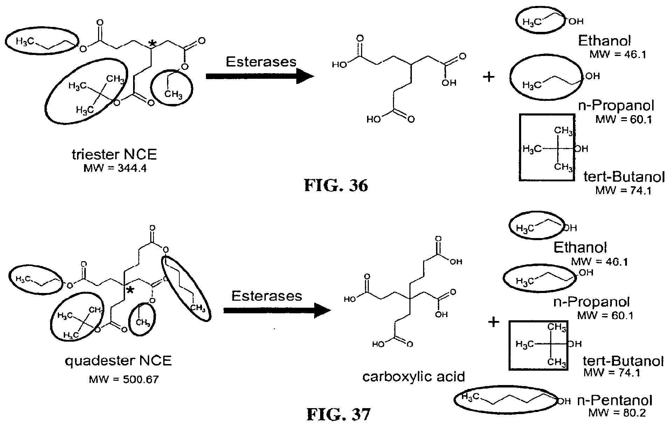

Below are nine examples (Figures 29-37) of how different Class 1, 2 and 3 molecules could be isotopically labeled for MAMS:
Ester Example 1: Aspartame - Figure 29. Aspartame is a food additive, considered GRAS by FDA; artificial sweetener. It mimics the taste of sugar in humans. It is rapidly metabolized by human gut esterases and gut peptidases in humans. Its metabolites consist of L-aspartic acid + L-Phenylalanine + Methanol. According to the subject invention, the NICE Embodiment - Chemical Group Site(s) of Isotopic Label(s) on Parent Molecular Structure: Preferred site is the methyl group on Aspartame (indicated by circle) but may include other locations on the parent molecule. In another embodiment of the invention, the NICE Embodiment - Type of Isotopic Labeling on Preferred Site(s): insertion of isotopic label(s) on the preferred site, including but not limited to a) a single label of a given isotope type (e.g., one Deuterium label = CDH2) on the preferred site(s), b) multiple labels of a given isotope (e.g., greater than one deuterium = CD2H or CD3) on the preferred site(s), or c) combinations of different types and numbers of isotopes (e.g., deuterium, carbon and/or oxygen = 13CDH2, 13CHD2, or 13CD3) on one or more locations of the preferred site(s). In yet another embodiment of the invention, the NICE Embodiment - Preferred Labeled Entity for Detection: isotopic (e.g., deuterium) labeled methanol in the breath; a less preferred embodiment would be labeled metabolic products of methanol (formaldehyde, formic acid and/or CO2 - see Figure 6 for details of metabolism of methanol). Isotopic labeling of larger metabolic fragments derived from the parent, which could be semi-volatile or non-volatile, could also serve as EDIMs, particularly if the liquid phase of breath is being analyzed.
Ester Example 2: Acetylsalicylic Acid - Figure 30. Acetylsalicylic Acid is an over the counter (OTC) drug. It is a nonsteroidal anti inflammatory drug (NSAID) that irreversibly inhibits cyclooxygenase (COX) via acetylation of the serine residue at the active site of COX, which suppresses production of prostaglandins and thromboxanes. It is metabolized by Acetylsalicylic Acid (ASA) esterases in humans. It metabolites consist of 2 acids (salicylic acid and acetic acid). In one embodiment, the NICE Embodiment - Chemical Group Site(s) of Isotopic Label(s) on Parent Molecular Structure: Preferred site is the methyl group on ASA (indicated by red circle) but may include other locations on the parent molecule. In another embodiment, the NICE Embodiment - Type of Isotopic Labeling on Preferred Site(s): NICE Embodiment - Type of Isotopic Labeling on Preferred Site(s): insertion of isotopic label(s) on the preferred site, including but not limited to a) a single label of a given isotope type (e.g., one Deuterium label = CDH2) on the preferred site(s), b) multiple labels of a given isotope (e.g., greater than one deuterium = CD2H or CD3) on the preferred site(s), or c) combinations of different types and numbers of isotopes (e.g., deuterium, carbon and/or oxygen = 13CDH2, 13CHD2, or 13CD3) on one or more locations of the preferred site(s). In yet another embodiment, the NICE Embodiment - Preferred Labeled Entity for Detection: isotopic (e.g., deuterium) labeled acetic acid in the breath; a less preferred embodiment would be labeled metabolic products of acetic acid, CO2 - see Figure 6 for details of metabolism of acetic acid). Isotopic labeling of larger metabolic fragments derived from the parent, which could be semi-volatile or non-volatile, could also serve as EDIMs, particularly if the liquid phase of breath is being analyzed.
Ester Example 3: Parabens - Figure 31. Paraben is an abbreviation for para-hydroxybenzoic acid. Parabens are a family of alkyl esters of para-hydroxybenzoic acid that differ at the para position of the benzene ring. There are four widely marketed para-hydroxybenzoic acid (POHBA) esters: methylparaben, ethylparaben, propylparaben, and butylparaben. Used as food additives/preservatives; considered GRAS by FDA; Europe uses as ADI (acceptable daily intake) up to 10 mg/kg per day for methyl and ethyl paraben. It inhibits bacterial growth; food additive. It is rapidly metabolized by carboxylesterases and tissue esterases in humans. Its metabolites consist of para-hydroxybenzoic acid (POHBA) + corresponding alcohol (see below for details). In one embodiment, the NICE Embodiment - Chemical Group Site(s) of Isotopic Label(s) on Parent Molecular Structure: Preferred site is the methyl group on Aspartame (indicated by red circle) but may include other locations on the parent molecule. In another embodiment, the NICE Embodiment - Type of Isotopic Labeling on Preferred Site(s): insertion of isotopic label(s) on the preferred site, including but not limited to a) a single label of a given isotope type (e.g., one Deuterium label = CDH2) on the preferred site(s), b) multiple labels of a given isotope (e.g., greater than one deuterium = CD2H or CD3) on the preferred site(s), or c) combinations of different types and numbers of isotopes (e.g., deuterium, carbon and/or oxygen = 13CDH2, 13CHD2, or 13CD3) on one or more locations of the preferred site(s). In yet another embodiment, the NICE Embodiment - Preferred Labeled Entity for Detection: isotopic (e.g., deuterium) labeled alcohols in the breath; a less preferable embodiment is labeled distal metabolic products of the alcohols and acids generated from the different parabens (see Figure 6 for details). Isotopic labeling of larger metabolic fragments derived from the parent, which could be semi-volatile or non-volatile, could also serve as EDIMs, particularly if the liquid phase of breath is being analyzed.
Ester Example 4: Clofibrate - Figure 32. Clofibrate is a prescription medication. It is a hypolipidemic drug, known to induce peroxisome proliferation; a member of a large class of diverse exogenous and endogenous chemicals known as peroxisome proliferators; Activation of the peroxisome proliferator activated receptor- (PPAR-α) key aspect of efficacy,. It is metabolized by human esterases. Its metabolites consist of carboxylic acid derivatives of Clofibrate + Ethanol. In one embodiment, the NICE Embodiment - Chemical Group Site(s) of Isotopic Label(s) on Parent Molecular Structure: Preferred site is the ethyl group on clofibrate, particularly on the methyl group (indicated by red circle) but may include other locations on the parent molecule. In another embodiment, the NICE Embodiment - Type of Isotopic Labeling on Preferred Site(s): insertion of isotopic label(s) on the preferred site, including but not limited to a) a single label of a given isotope type (e.g., one Deuterium label = CH2CH2D) on the preferred site(s), b) multiple labels of a given isotope (e.g., greater than one deuterium = CH2CHD2, CH2D3, CHDCD3, CD2CD3) on the preferred site(s), or c) combinations of different types and numbers of isotopes (e.g., deuterium, carbon and/or oxygen on one or more locations of the preferred site(s). In yet another embodiment, the NICE Embodiment - Preferred Labeled Entity for Detection: isotopic (e.g., deuterium-based) labeled ethanol in the breath; a less preferred embodiment would be labeled metabolic products of ethanol (acetaldehyde, acetic acid and/or CO2 - see Figure 6 for details of metabolism of ethanol). Isotopic labeling of larger metabolic fragments derived from the parent, which could be semi-volatile or non-volatile, could also serve as EDIMs, particularly if the liquid phase of breath is being analyzed.
Ester Example 5: Esmolol - Figure 33. Esmolol is a controlled/prescription drug. It is an ester-based ultra short acting beta blocker that is beta1 receptor selective. In contrast to most ester-containing drugs, the hydrolysis of esmolol is mediated by an esterase in the cytosol of red blood cells (RBC) called arylesterase. Its metabolites consist of carboxylic acid derivatives of Esmolol + Methanol. In one embodiment, the NICE Embodiment - Chemical Group Site(s) of Isotopic Label(s) on Parent Molecular Structure: Preferred site is the methyl group on esmolol (indicated by red circle) but may include other locations on the parent molecule. In another embodiment, the NICE Embodiment - Type of Isotopic Labeling on Preferred Site(s): NICE Embodiment - Type of Isotopic Labeling on Preferred Site(s): insertion of isotopic label(s) on the preferred site, including but not limited to a) a single label of a given isotope type (e.g., one Deuterium label = CDH2) on the preferred site(s), b) multiple labels of a given isotope (e.g., greater than one deuterium = CD2H or CD3) on the preferred site(s), or c) combinations of different types and numbers of isotopes (e.g., deuterium, carbon and/or oxygen = 13CDH2, 13CHD2, or 13CD3) on one or more locations of the preferred site(s). In yet another embodiment, the NICE Embodiment - Preferred Labeled Entity for Detection: isotopic (e.g., deuterium) labeled methanol in the breath; a less preferred embodiment would be labeled metabolic products of methanol (formaldehyde, formic acid and/or CO2 - see Figure 6 for details of metabolism of methanol). Isotopic labeling of larger metabolic fragments derived from the parent, which could be semi-volatile or non-volatile, could also serve as EDIMs, particularly if the liquid phase of breath is being analyzed.
Ester Example 6: Procaine - Figure 34. Procaine is a prescription drug. It is a local anesthetic for nerve conduction blocks; blocks sodium (Na+) channels. It is metabolized in humans by human pseudocholinesterase (butyrylcholinesterase). Its metabolites consist of carboxylic acid derivatives of procaine (para-aminobenzoic acid) + 2-(Diethylamino)-ethanol. In one embodiment, the NICE Embodiment - Chemical Group Site(s) of Isotopic Label(s) on Parent Molecular Structure: Preferred site is one or both ethyl groups on procaine, preferentially located on the methyl (ethyl group indicated by red circle) but may include other locations on the parent molecule. In another embodiment, the NICE Embodiment - Type of Isotopic Labeling on Preferred Site(s): insertion of isotopic label(s) on the preferred site, including but not limited to a) a single label of a given isotope type (e.g., one Deuterium label = CH2CH2D) on the preferred site(s), b) multiple labels of a given isotope (e.g., greater than one deuterium = CH2CHD2, CH2D3, CHDCD3, CD2CD3) on one or two ethyl groups as the preferred site(s), or c) combinations of different types and numbers of isotopes (e.g., deuterium, carbon, nitrogen and/or oxygen on one or more locations of the preferred site(s). In yet another embodiment, the NICE Embodiment - Preferred Labeled Entity for Detection: isotopic (e.g., deuterium-based) labeled 2-(Diethylamino)-ethanol in the breath. Isotopic labeling of larger metabolic fragments (e.g., PABA) derived from the parent, which could be semi-volatile or non-volatile, could also serve as EDIMs, particularly if the liquid phase of breath is being analyzed.
Ester Example 7: New Chemical Entity - Cyclic Molecule Containing 3 Ester Bonds - Figure 35. Figure 35 illustrates an ester-based cyclic NCE that can generate 3 different alcohols (ethanol, n-propanol, and tert-butanol) as EDIMs. Each of the 3 ester bonds on the NCE will be hydrolyzed and release a carboxylic acid(s) and 3 different alcohols. At least 3 major advantages exist for creating these types of NCEs for MAMS applications: 1) it allows custom-designed patterns of EDIMs to be released via enzymes (either a single type or more than one type) that can provide excellent discrimination (e.g., combination of EDIMs in breath is highly distinctive and can be used to eliminate the effect of diet or disease on MAMS function), 2) different combinatorial permutations (e.g., a single NCE containing one or more ester groups versus combinations of different NCEs, each containing one or more distinctive ester groups) of these types of molecules can be used to isotopically and/or non-isotopically label different dosage forms of a given drug (e.g., warfarin) and/or multiple types of different medications, and 3) by combining distinctive EDIMs into a NCE, the mass of drug (preferred embodiment is the solid form) required to release these patterns of EDIMs can be minimized. The latter is important because mass limitations in a pill matrix for MAMS will exist. For example, for the NCE shown above (MW=336.4), approximately 54% of it's mass will liberate the mass of 3 different alcohols. Contrast this to another agent that would generate ethanol by esterases, ethyl paraben (only 28% of it's mass will liberate the mass of 1 alcohol, ethanol); it has a much lower mass efficiency to generate the EDIM. In addition, the larger size of these more mass efficient molecules will likely have a solid physical state, which simplifies integration into the MAMS pill system. Of course, the maximum EDIM concentration in the breath will be primarily dependent upon the mass of NCE, the intrinsic rate of generation of EDIM by enzyme(s), and the physiochemical characteristics of the EDIMs in the body. The isotopic labels shown in Table 2, preferably but not limited to deuterium, can be used to label various atoms of the NCE, which in turn, will generate a wide array of isotopically (approach described in Figures 29-34) and/or non-isotopically labeled alcohols that will serve as EDIMs in this example. In addition, Isotopic labeling of larger metabolic fragments derived from the parent (e.g., carboxylic acid in this embodiment), which could be semi-volatile or non-volatile, could also serve as EDIMs, particularly if the liquid phase of breath is being analyzed. Note: the number of carbon linkages between the ring structure and the carbonyl bond can be varied to optimize the molecular properties of the molecule.
Ester Example 8: New Chemical Entity - Non-Cyclic Molecule Containing 3 Ester Bonds - Figure 36. Figure 36 illustrates an ester-based non-cyclic NCE that can generate 3 different alcohols (ethanol, n-propanol, and tert-butanol) as EDIMs. Each of the 3 ester bonds on the NCE will be hydrolyzed and release a carboxylic acid(s) and 3 different alcohols. At least 3 major advantages exist for creating these types of NCEs for MAMS applications: 1) it allows custom-designed patterns of EDIMs to be released via enzymes (either a single type or more than one type) that can provide excellent discrimination (e.g., combination of EDIMs in breath is highly distinctive and can be used to eliminate the effect of diet or disease on MAMS function), 2) different combinatorial permutations (e.g., a single NCE containing one or more ester groups versus combinations of different NCEs, each containing one or more distinctive ester groups) of these types of molecules can be used to label different dosage forms of a given drug (e.g., warfarin) and/or multiple types of different medications, and 3) by combining distinctive EDIMs into a NCE, the mass of drug (preferred embodiment is the solid form) required to release these patterns of EDIMs can be minimized. The latter is important because mass limitations in a pill matrix for MAMS will exist. For example, for the NCE shown above (MW=344.4), approximately 52% of it's mass will liberate the mass of 3 different alcohols. Contrast this to another agent that would generate ethanol by esterases, ethyl paraben (only 28% of it's mass will liberate the mass of 1 alcohol, ethanol); it has a much lower mass efficiency to generate the EDIM. In addition, the larger size of these more mass efficient molecules will likely have a solid physical state, which simplifies integration into the MAMS pill system. Of course, the maximum EDIM concentration in the breath will be primarily dependent upon the mass of NCE, the intrinsic rate of generation of EDIM by enzyme(s), and the physiochemical characteristics of the EDIMs in the body. The isotopic labels shown in Table 2, preferably but not limited to deuterium, can be used to label various atoms of the NCE, which in turn, will generate a wide array of isotopically (approach described in Figures 29-34) and/or non-isotopically labeled alcohols that will serve as EDIMs in this example. In addition, Isotopic labeling of larger metabolic fragments derived from the parent (e.g., carboxylic acid in this embodiment), which could be semi-volatile or non-volatile, could also serve as EDIMs, particularly if the liquid phase of breath is being analyzed. Note: the number of carbon linkages between the central carbon (indicated by asterisk) and the carbonyl bond can be varied to optimize the molecular properties of the molecule.
Ester Example 9: New Chemical Entity - Non-Cyclic Molecule Containing 4 Ester Bonds - Figure 37. Figure 37 illustrates an ester-based non-cyclic NCE that can generate 4 different alcohols (ethanol, n-propanol, tert-butanol, n-pentanol) as EDIMs. Each of the 4 ester bonds on the NCE will be hydrolyzed and release a carboxylic acid(s) and 4 different alcohols. At least 3 major advantages exist for creating these types of NCEs for MAMS applications: 1) it allows custom-designed patterns of EDIMs to be released via enzymes (either a single type or more than one type) that can provide excellent discrimination (e.g., combination of EDIMs in breath is highly distinctive and can be used to eliminate the effect of diet or disease on MAMS function), 2) different combinatorial permutations (e.g., a single NCE containing one or more ester groups versus combinations of different NCEs, each containing one or more distinctive ester groups) of these types of molecules can be used to label different dosage forms of a given drug (e.g., warfarin) and/or multiple types of different medications, and 3) by combining distinctive EDIMs into a NCE, the mass of drug (preferred embodiment is the solid form) required to release these patterns of EDIMs can be minimized. The latter is important because mass limitations in a pill matrix for MAMS will exist. For example, for the NCE shown above (MW=500.67), approximately 52% of it's mass will liberate the mass of 4 different alcohols. Contrast this to another agent that would generate ethanol by esterases, ethyl paraben (only 28% of it's mass will liberate the mass of 1 alcohol, ethanol); it has a much lower mass efficiency to generate the EDIM. In addition, the larger size of these more mass efficient molecules will likely have a solid physical state, which simplifies integration into the MAMS pill system. Of course, the maximum EDIM concentration in the breath will be primarily dependent upon the mass of NCE, the intrinsic rate of generation of EDIM by enzyme(s), and the physiochemical characteristics of the EDIMs in the body. The isotopic labels shown in Table 2, preferably but not limited to deuterium, can be used to label various atoms of the NCE, which in turn, will generate a wide array of isotopically (approach described in Figures 29-34) and/or non-isotopically labeled alcohols that will serve as EDIMs in this example. In addition, Isotopic labeling of larger metabolic fragments derived from the parent (e.g., carboxylic acid in this embodiment), which could be semi-volatile or non-volatile, could also serve as EDIMs, particularly if the liquid phase of breath is being analyzed. Note: the number of carbon linkages between the central carbon (indicated by asterisk) and the carbonyl bond can be varied to optimize the molecular properties of the molecule.

### CYP450 Enzymes

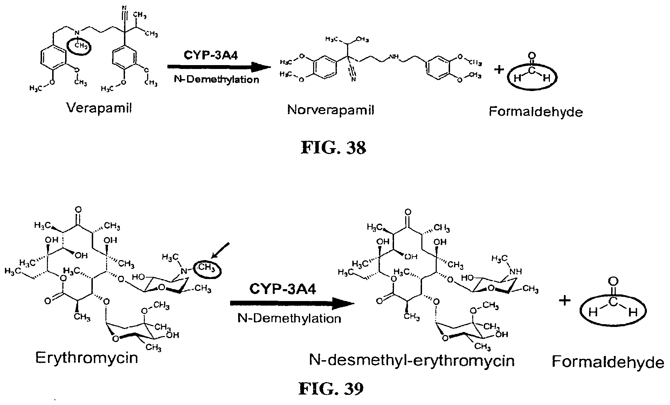

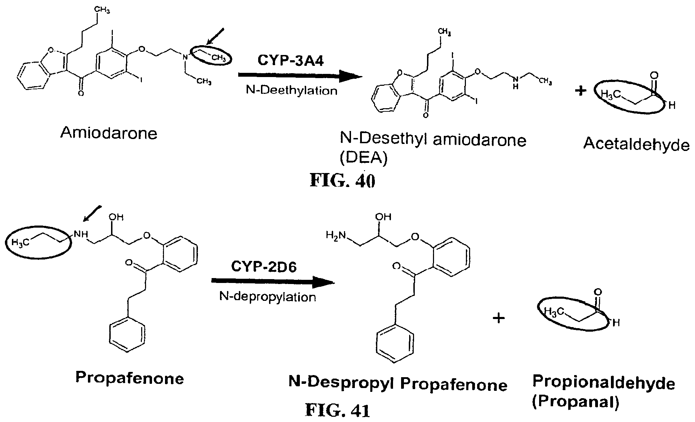

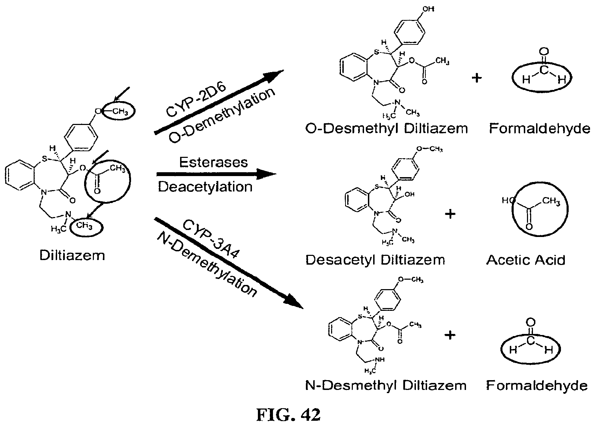

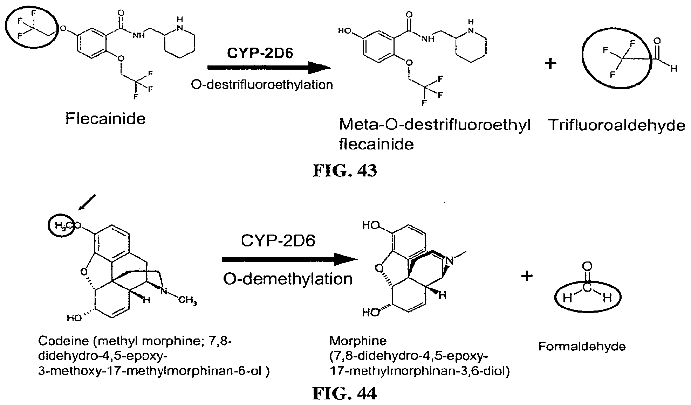

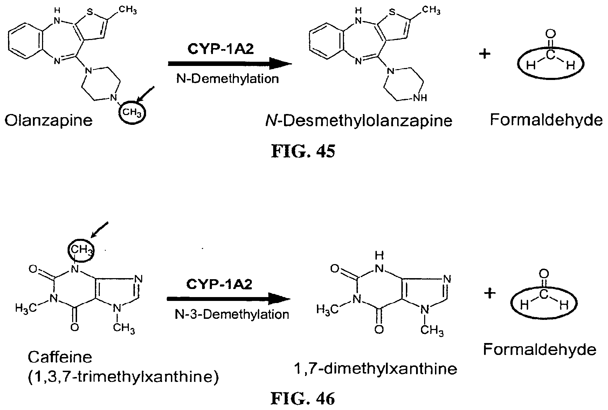

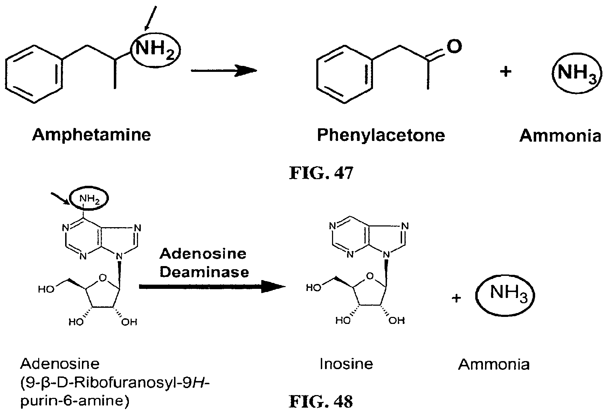

Although many enzymatic systems biotransform drugs in humans, the most important and versatile one is the cytochrome P450 mixed function oxidase (MFO) system, especially for lipophilic xenobiotics. It is a remarkable system that has it roots of origin over 3.5 billion years ago. The CYP system is a heme containing, molecular oxygen requiring, membrane bound system containing over 160 known members. A reduced cofactor, NADPH⁺, and a coenzyme, cytochrome P450 NADPH oxidoreductase, are critical for P450 activity, whereas a membrane bound hemoprotein, cytochrome b5, can further stimulate P450 catalytic activity, most notably for the 3A family. NADPH oxidoreductase transfers electrons from NADPH to the various isoforms of P450. The level of these factors can markedly affect the activity of the CYP components. P450 is primarily synthesized and located in the liver, but other production and location sites (e.g., small intestine, kidney) are known to exist. Hepatic P450 is located in the endoplasmic reticulum and mitochondria. It plays a major role in the metabolism of numerous physiological substrates such as prostaglandins, steroids, bile acids plus a large number of clinically important drugs. The CYP system is responsible for the reduction, oxidation and hydrolysis of lipophilic drugs. The two major CYP enzymes, CYP3A4 and CYP2D6 catalyze dealkylation, hydroxylation, dehalogenation, dehydration, and nitroreduction reactions. By incorporating various isotopic labels listed in Table 2 (preferred embodiment is deuterium) into the various atomic sites of the CYP450 substrates (e.g., including but not limited to deuterium for ordinary hydrogen; ¹⁷O and/or ¹⁸O for ordinary oxygen, or ¹³C for ordinary carbon, where appropriate), various EDIMs (arising from the CYP substrate, aldehyde, acid and CO₂) containing one or more isotopic labels could be generated that will fulfill the requirements of an effective MAMS. Shown below are ten examples of CYP450 substrates (Figures 38-47), which are FDA approved or GRAS-type drugs that could be isotopically labeled for an effective MAMS, and in some cases to create "smart" therapeutic agents:
CYP Substrate Example 1 - Enzyme: CYP-3A4 - Substrate: Verapamil - Figure 38. Verapamil (2,8-bis-(3,4-dimethoxyphenyl)-6-methyl-2-isopropyl-6-azaoctanitrile) is a L-type calcium channel blocker that liberates formaldehyde upon oxidative dealkylation (N-demethylation) by CYP-3A4. Orally administered verapamil undergoes extensive metabolism in the liver. One major metabolic pathway is the formation of norverapamil (N-methylated metabolite of verapamil) and formaldehyde by CYP-3A4. Although dependent upon the number of alternate metabolic pathways, the rate of formation of a specific metabolite(s) (i.e., verapamil → norverapamil and formaldehyde via CYP-3A4) generally appears to be predictive of *in vivo* functional enzyme competence. In fact verapamil is metabolized by O-demethylation (25%) and N-dealkylation (40%). The CYP-3A4 is most the important enzyme in humans for metabolizing drugs. It has been estimated that the CYP-3A4 isoform of the P450 system is responsible for metabolizing 55-60% of all pharmaceutical agents. The CYP3A4 plays a critical role in metabolizing many drugs, including several cytotoxic drugs such as paclitaxel, docetaxel, vinorelbine, vincristine, irinotecan, topotecan, ifosfamide, cyclophosphamide, and tamoxifen. Thus, alterations in CYP-3A4 function frequently lead to drug-induced increases in morbidity and mortality. The isotopic labels shown in Table 2 (preferably deuterium), where appropriate, can be used to label various atoms (red circle) of verapamil, which in turn, will generate isotopic-labeled formaldehyde that will serve as the preferred embodiment of the EDIM in this example. In addition, isotopic labeling of larger metabolic fragments (e.g., norverapamil, etc.) derived from the parent, which could be semi-volatile or non-volatile, could also serve as EDIMs, particularly if the liquid phase of breath is being analyzed.
CYP Substrate Example 2 - Enzyme: CYP-3A4 - Substrate: Erythromycin - Figure 39. Erythromycin is a macrolide antibiotic, which prevents protein synthesis in bacteria, and is thus used to treat various infections, particularly in patients who are allergic to penicillin. Because erythromycin is also a potent motolin agonist, it markedly enhances gastric emptying. This gastrokinetic action is known to wane in a short period of time, due to the development of tachyphylaxis/desensitization. The erythromycin breath test (EBT) is used to assess CYP-3A4 function. Erythromycin is *N*-demethylated by CYP-3A4, and the cleaved methyl group is released as formaldehyde and, eventually, as formic acid then CO2. The test is performed by intravenously administering a trace amount of 14C labeled erythromycin and then measuring the amount of exhaled 14CO2. The rate of release of 14CO2 in expired breath is thought to reflect hepatic CYP3A4 activity. The isotopic labels shown in Table 2 (preferably deuterium), where appropriate, can be used to label various atoms (red circle) of erythromycin, which in turn, will generate isotopic-labeled formaldehyde that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 3 - Enzyme: CYP-3A4 - Substrate: Amiodarone - Figure 40. Amiodarone is one of the most effective antiarrhythmic drugs in clinical medicine. It is highly effective in treating atrial fibrillation, particularly in preventing its re-occurrence. Although this drug has a complex mechanistic profile (blocks sodium channels, beta receptors, calcium channels, and potassium channels) its major electrophysiological action is to prolong repolarization in cardiac tissue, predominantly by blocking potassium channels. Therefore, it is classified as a Class III antiarrythmic drug according to the Vaughn-William Classification. The isotopic labels shown in Table 2 (preferably deuterium), where appropriate, can be used to label various atoms (red circle) of amiodarone, which in turn, will generate isotopic-labeled acetaldehyde that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 4 - Enzyme: CYP-3A4 - Substrate: Propafenone - Figure 41. Propafenone is an antiarrhythmic drug that acts by primarily blocking sodium channels, and is classified as a Class IC antiarrythmic drug according to the Vaughn-William Classification. The isotopic labels shown in Table 2 (preferably deuterium), where appropriate, can be used to label various atoms (red circle) of propafenone, which in turn, will generate isotopic-labeled propionaldehyde that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 5 - Enzymes: CYP-3A4 + CYP-2D6 + Esterase - Substrate: Diltiazem - Figure 42. Diltiazem is a L-type calcium channel blocker, which undergoes complex biotransformation, including deacetylation, *N*-demethylation, and *O*-demethylation. Of these pathways, CYP-3A4 probably plays a more prominent role than CYP2D6 in the metabolism of diltiazem. The isotopic labels shown in Table 2 (preferably deuterium), where appropriate, can be used to label various atoms (red circle) of diltiazem, which in turn, will generate isotopic-labeled formaldehyde and/or acetic acid that will serve as the preferred embodiments of the EDIMs in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 6 - Enzyme: CYP-2D6 - Substrate: Flecainide - Figure 43. Flecainide is an antiarrhythmic drug that acts by primarily blocking sodium channels, and is classified as a Class IC antiarrythmic drug according to the Vaughn-William Classification. Flecainide is characterized as a unique drug, given its high content of fluorine. CYP-2D6 liberates a highly distinctive, volatile fluorinated aldehyde metabolite, termed trifluoroaldehyde. The isotopic labels shown in Table 2 (preferably non-ordinary carbon), where appropriate, can be used to label various atoms (red circle) of flecainide, which in turn, will generate isotopic-labeled trifluoroaldehyde that will serve as the preferred embodiment of the EDIM in this example. Note: the unique nature of fluorinated aldehyde will likely allow a MAMS to be constructed without the need for isotopic labeling in the case of flecainide. In addition, isotopic labeling of larger metabolic fragments derived from the parent, which could be semi-volatile or non-volatile, could also serve as EDIMs, particularly if the liquid phase of breath is being analyzed.
CYP Substrate Example 7 - Enzyme: CYP-2D6 - Substrate: Codeine - Figure 44. Shown is an example where the CYP substrate is a prodrug (codeine) that is converted by the P450 system (CYP 2D6) into the active drug (morphine). Morphine has a significantly higher affinity for the µ opioid receptor than codeine, and thus is thought to mediate the analgesic properties of codeine. Only about 10% of codeine is normally converted to morphine in vivo. In this embodiment, the NICE system could be used to not only ensure that codeine is efficacious (i.e., ensures adequate conversion to morphine) but also to ensure that an inordinate amount of codeine isn't converted to morphine if a subject has a super functional of CYP-2D6. The latter scenario would cause an adverse drug reaction (ADR) because an excessive amount of morphine would be present in the body. Likewise, in the former scenario, the NICE system would identify those subjects that wouldn't get adequate pain relief from this drug, because not enough morphine is produced from codeine. The function of CYP 2D6 is altered by a great many factors including but not limited to genetics or drug-drug interactions. For example, because 6-10% of Caucasians have poorly functional CYP2D6, they do not get adequate pain relief from codeine. Furthermore, a number of medications are potent CYP2D6 inhibitors and reduce or even completely eliminate the efficacy of codeine. The most notorious of these are the SSRIs including fluoxetine (Prozac) and citalopram (Celexa). The high end PO dose of codeine is typically 240 mg given over 24 hours. The small arrow indicates the site of catalytic action by the CYP enzyme to liberate the formaldehyde. The isotopic labels shown in Table 2 (preferably deuterium), where appropriate, can be used to label various atoms (red circle) of codeine, which in turn, will generate isotopic-labeled formaldehyde that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 8 - Enzyme: CYP-1A2 - Substrate: Olanzapine - Figure 45. Olanzapine is one of the most widely used antipsychotic drugs in the world. It is used to treat schizophrenia. The major metabolic pathway for olanzapine is mediated by CYP-1A2. Its metabolism is well predicted by using the caffeine breath test as a probe to examine the ability of the CYP450 system to metabolism olanzapine. The small arrow indicates the site of catalytic action by the CYP enzyme to liberate the formaldehyde. The isotopic labels shown in Table 2 (preferably deuterium), where appropriate, can be used to label various atoms (red circle) of olanzapine, which in turn, will generate isotopic-labeled formaldehyde that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 9 - Enzyme: CYP-1A2 - Substrate: Caffeine - Figure 46. Caffeine is a xanthine-type drug that is widely found in many foods, including beverages. Caffeine is a central nervous stimulant. It has been generally accepted as a specific *in vivo* probe for CYP1A2 activity. Approximately 80% of caffeine given orally to humans is converted to theophylline. Caffeine has been shown to provide an accurate phenotypic probe for measuring CYP1A2 activity, particularly when predicting the ability of olanzapine to be metabolized in vivo. The small arrow indicates the site of catalytic action by the CYP enzyme to liberate the formaldehyde. The isotopic labels shown in Table 2 (preferably deuterium), where appropriate, can be used to label various atoms (red circle) of caffeine, which in turn, will generate isotopic-labeled formaldehyde that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.
CYP Substrate Example 10 - Enzyme: CYP-2C - Substrate: Amphetamine - Figure 47. Amphetamine (alpha-methyl-phenethylamine) is a central nervous system (CNS) stimulant used primarily to treat attention-deficit hyperactivity disorder (ADHD) and narcolepsy. Unfortunately, the drug is widely used recreationally as a club drug and as a performance enhancer, and humans can be become highly addicted to this drug. The small arrow indicates the site of catalytic action by the CYP enzyme (CYP-2C) to liberate the ammonia via deamination. The isotopic labels shown in Table 2 (preferably non-ordinary nitrogen and to a lesser degree deuterium), where appropriate, can be used to label various atoms (red circle) of amphetamine, which in turn, will generate isotopic-labeled ammonia that will serve as the preferred embodiment of the EDIM in this example. In addition, isotopic labeling of larger metabolic fragments derived from the parent, which could be semi-volatile or non-volatile, could also serve as EDIMs, particularly if the liquid phase of breath is being analyzed.

From the perspective of preventing ADRs and monitoring enzyme competency, there is one aspect of the CYP enzyme system that can be exploited. Most drugs are metabolized by oxidative N-dealkylation. It is commonly observed that the alkyl group lost from an amine during N-dealkylation (and from an ether during O-dealkylation) appears as an aldehyde or ketone arising from the dissociation of a carbinolamine intermediate. Aldehydes and ketones are volatile, so deuteration (or other form of isotopic labeling) of medications on the portion of the molecule that forms the aldehyde or ketone will result in a "reporter" that is volatile and will appear in the breath

### Deaminases - Adenosine Deaminase

Adenosine deaminase (also known as ADA) is an enzyme (EC 3.5.4.4) involved in purine metabolism. It very rapidly metabolizes the nucleoside adenosine ingested from food and/or produced from turnover of nucleic acids in tissues. Adenosine is an FDA approved drug for intravenous use in treating supraventricular tachyarrhythmias involving the AV node (via activation of A₁ receptors that depress nodal conduction) and for improving the quality of cardiac perfusion scans (via A₂ receptor-mediated dilation of coronary vessels). By removing an amine group, adenosine deaminase irreversibly deaminates adenosine to the related nucleoside, inosine. Inosine, in turn, can be deribosylated (removed from ribose) by another enzyme called purine nucleoside phosphorylase (PNP), converting it to hypoxanthine.

Figure 48 is illustrative of the above. Adenosine is a nucleoside, which is naturally found in the body, that is highly effective when given intravenously at treating reentrant supraventricular tachyarrhythmias involving the AV node as part of the reentrant circuit. By activating A1 receptors and increasing IKADO conductance, adenosine effectively terminates these rhythm disorders by profound depressing AV nodal conduction. Due to the rapid degradation by adenosine deaminase, which is ubiquitous in the body, orally administered adenosine used for MAMS should effectively liberate ammonia without incurring a significant increase in plasma adenosine levels in the blood. The small arrow indicates the site of catalytic action by the CYP enzyme to liberate the ammonia. The isotopic labels shown in Table 2 (preferably non-ordinary nitrogen or deuterium), where appropriate, can be used to label various atoms (red circle) of adenosine, which in turn, will generate isotopic-labeled ammonia that will serve as the preferred embodiment of the EDIM in this example. In addition, isotopic labeling of larger metabolic fragments (e.g., inosine) derived from the parent, which could be semi-volatile or non-volatile, could also serve as EDIMs, particularly if the liquid phase of breath is being analyzed.

### Implication in Drug Development of Using Isotopic Labeled Therapeutic Entities: A Summary of the Concept of "NICE" (New Intelligent Chemical Entity)

In this patent application, technologies are described that potentially create a new area in drug design research and development - the advent of "smart" or "intelligent" therapeutic agents. What is the definition of a "smart" drug? When humans use therapeutic agents to treat various medical disorders, two major limitations make these entities have suboptimal efficacy and safety: 1) non-adherence (patients don't take the drugs as instructed by their health care provider in terms of dose and/or frequency), and/or 2) adverse drug reactions (ADRs) resulting from but not limited to drug-drug interactions (DDIs) or genetic defects (e.g., genetic polymorphisms in CYP enzymes including but not limited to 2D6, 2C9, 2C19; genetic polymorphism of vitamin K epoxide reductase [VKORC1] involving warfarin therapy). A "smart" therapeutic agent of the invention is designed to reliably and accurately "self report" key elements of its safety and efficacy during chronic therapy by incorporating 3 types of functions into a medication system:
**1:** continuously documenting a particular therapeutic agent was administered (preferable embodiment is ingestion of pill via oral route) at the right time intervals (frequency), hereafter termed **F_{Freq}**
**2:** continuously documenting a specific dose of a particular therapeutic agent was administered in the proper amount (dose), hereafter termed **F_{Dose}**
**3:** continuously documenting a particular therapeutic agent was being properly metabolized, hereafter termed **F_{Metab}**

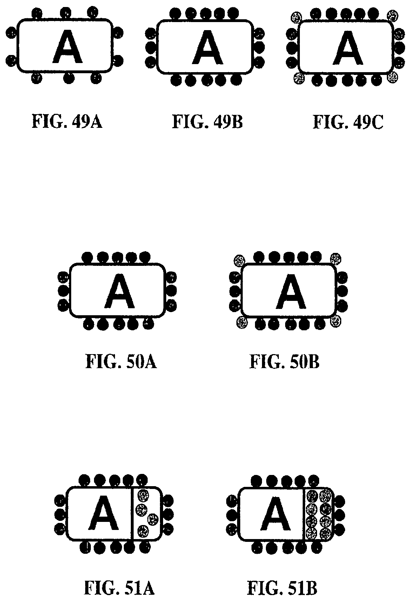

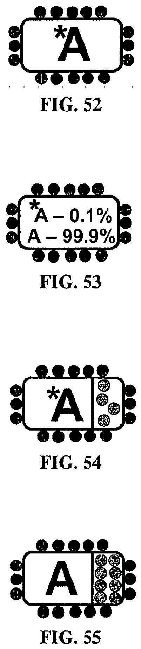

By specifically engineering these functional attributes into a therapeutic agent, it would not only make pharmaceutical therapies safer and more efficacious, but also create new medications from either existing drugs (generic and/or on patent) with minimal-to-no new regulatory issues or create easy pathways to design and synthesize new molecular entities that have these beneficial functional attributes incorporated into the system from the inception of the molecule. These types of therapeutic agents, hereafter termed NICE (New "Intelligent" Chemical Entity)-type molecules, would represent a new paradigm in drug discovery and development. Unfortunately, thousands of drugs, both generic and patented, currently on the market being sold to consumers are not intelligent, but in fact are "dumb". That is, they provide no continuous feedback to patients and/or health care providers as to their efficacy and safety, which is particularly important when medications are given over sustained periods of time to treat a variety of diseases. With the current invention, generic or patented "dumb" drugs already on the market could be educated and made intelligent without incurring the liability of new regulatory hurdles, or alternately, new molecular entities being developed for different disease could be designed to be intelligent right from the start. The ideal NICE-type therapeutic agents would be trifunctional **(F_{Freq}F_{Dose}F_{Metab})** in nature. In fact, having all 3 attributes would make them "genius" molecules. However, a number of different combinations of the 3 individual functions (F_{Freq}, F_{Dose}, and/or F_{Metab}) could be incorporated into a NICE system; therefore embodiments of the NICE system could be bifunctional or even monofunctional in nature. Figures 49 to 59 provides details and teaches how to construct these different types of "smart" medications systems, ranging from the most simple NICE-type medications (Figure 49) to among the most complex (Figure 55). In another embodiment, a fourth element called the therapeutic drug monitoring (TDM) function, termed the **F_{TDM}**, could also be integrated into a medication system. For example, in one embodiment F_{TDM} could be integrated into F_{Freq}, F_{Dose}, and F_{Metab} to create a quad-functional "smart" pill system: **F_{Freq}F_{Dose}F_{Metab}F_{TDM}**. F_{TDM} indicates the ability of a smart pill system to measure the concentration of the active therapeutic drug (A) in the blood, using a surrogate concentration of A in the breath, preferably using the liquid phase of breath. In this embodiment A may or may not be isotopically labeled (preferably with deuterium, see Table 2 for additional options).

In Figure 49A, a single taggant (Tcircles) is on pill surface containing the active therapeutic agent, A (which is not labeled). The **EDIM** is **Tcircles** (i.e., not a metabolite of T, T1circles). When placed in the mouth, a pill surface-derived EDIM (Tcircles) is immediately liberated and will activate a sensor (indicates detection of Tcircles), which is preferably portable and hand held, when a sample of exhaled breath is provided to the sensor. With this embodiment, immediate notification of pill ingestion and simplicity of system are provided.

In Figure 49B, two taggants (Tgray and Tblack) are on pill surface containing the active therapeutic agent, A. The **EDIMs** are **Tgray** and **Tblack** (i.e., not a metabolite of Tgray, T1gray; not a metabolite of Tblack , T1black). When placed in the mouth, two pill surface-derived EDIMs (Tgray and Tblack) are immediately liberated and will activate a sensor (indicates detection of Tgray and/or Tblack) when exhaled into. With this embodiment, immediate notification of pill ingestion and simplicity of system are provided. Further, chance of interference of EDIM detection by various factors (e.g., diet, metabolism, disease) is very low if Tgray and Tblack are simultaneously detected in breath. Note: In this figure different combinations of surface taggants (e.g., different pairs, different triads could be used to label different doses of a given drug or different drugs.

In Figure 49C, three taggants (Tdarkgray, Tlightgray, Tblack) are on pill surface containing the active therapeutic agent, A. The **EDIMs** are **Tdarkgray, Tlightgray, Tblack** (i.e., not a metabolite of Tdarkgray, T1darkgray; not a metabolite of Tlightgray, T1lightgray; not a metabolite of Tblack , T1black). When placed in the mouth, three pill surface-derived EDIMs (Tdarkgray, Tblack, Tlightgray) are immediately liberated and will activate a sensor (indicating detection of Tdarkgray, Tblack, and/or Tlightgray) when exhaled into. With this embodiment, immediate notification of pill ingestion and simplicity of system. Further, chance of interference of EDIM detection in breath by various factors (e.g., diet, metabolism, disease) is virtually impossible if Tdarkgray, Tlightgray, and Tblack are simultaneously detected in breath.

In Figure 50A, two taggants (Tgray and Tblack) are on pill surface containing the active therapeutic agent, A. The **EDIMs** are **Tgray, Tblack,** and a metabolite of Tgray, **T1gray.** When placed in the mouth, two pill surface-derived EDIMs (Tgray and Tblack) are immediately liberated and will activate a sensor when exhaled into shortly (detection of Tgray and Tblack) after ingesting the pill; later when Tgray enters the gastrointestinal tract (GIT) and is absorbed into the blood, it is metabolized to T1gray that will appear in the breath and activate the sensor when exhaled into (detection of T1gray). In this embodiment, immediate notification of pill placement in mouth and confirmation of A entering the blood (pill entering GIT and being absorbed) is provided. One taggant (Tgray) provides dual functionality in this embodiment: 1) immediate confirmation of putting the pill in the mouth while 2) confirming the therapeutic drug actually entered the blood. Allows flexibility of confirming medication adherence on the basis of using either an early breath (Tgray and Tblack), a later breath (T1gray), or both. Literally guarantees ingestion of A. Very low chance of interference of EDIM detection by various factors (e.g., diet, metabolism, disease).

In Figure 50B, three taggants (Tdarkgray, Tlightgray, Tblack) are on pill surface containing the active therapeutic agent, A. The **EDIMs** are **Tdarkgray, Tlightgray, Tblack** and metabolite of Tblack, **T1black.** Alternately, in this embodiment, it is not critical that Tblack be an EDIM, since the combination of Tdarkgray and Tlightgray will still provide excellent discrimination from breath interferants. When placed in the mouth, three pill surface-derived EDIMs (Tdarkgray, Tlightgray, Tblack) are immediately liberated and will activate a sensor (detection of Tdarkgray, Tlightgray, Tblack) when exhaled into after ingesting the pill; later when Tblack enters the GIT and is absorbed into the blood, it is metabolized to T1black that will appear in the breath and activate the sensor when exhaled into (detection of T1black). In this embodiment, the addition of the 3rd taggant (Tblack) provides extra EDIM discrimination that the pill was placed into the mouth. Interference of EDIM measurements by various factors (e.g., diet, metabolism, disease) is virtually impossible if Tdarkgray, Tlightgray, and Tblack are simultaneously detected in breath. Note: In this figure different combinations of surface taggants (e.g., different pairs, different triads could be used to label different doses of a given drug or different drugs.

In Figure 51A, two taggants (Tdarkgray and Tblack) located on the surface and one taggant (Tlightgray) is placed inside the pill in a manner that makes it physically distinct from the active therapeutic agent, A. The **EDIMs** are **Tdarkgray, Tblack,** and a metabolite of Tlightgray, **T1lightgray.** When placed in mouth, two pill surface-derived EDIMs (Tdarkgray and Tblack) are immediately liberated and will activate a sensor when exhaled into shortly (detection of Tdarkgray and Tblack) after ingesting the pill; later when Tlightgray enters the GIT and is absorbed into the blood, it is metabolized to Tllightgray that will appear in the breath and activate the sensor when exhaled into (detection of T1lightgray). This embodiment is similar to that of Figure 50B, wherein this embodiment provides immediate notification of pill placement in mouth and confirmation of A entering the blood (pill entering the GIT and being absorbed). One taggant (Tlightgray) serves only to indicate that the pill contents entered the blood. Its placement inside the pill (versus on the surface - as illustrated in Figure 50B) makes Tlightgray a more efficient source of T1lightgray (more reliable delivery to GIT and blood entry), and hence improves the quality of MAMS. Note: In this figure different combinations of surface taggants (e.g., different pairs analogous to Tdarkgray and Tblack) could be used to label different doses of a drug or different drugs.

In Figure 51B, two taggants (Tdarkgray, Tblack,) are on the pill surface and two taggants (Tlightgray1, Tlightgray2) are within the pill containing the active therapeutic agent, A. The **EDIMs** are **Tdarkgray, Tblack,** metabolite of Tlightgray1, **T1lightgray1** and metabolite of Tlightgray2, **T1lightgray2.** This embodiment is nearly identical to that of Figure 51A except one additional taggant (Tlightgray2) was added that generates a second metabolite-based EDIM (T1lightgray2) in addition to T1lightgray1 that confirms the pill contents entered the GIT and subsequently the blood. Thus, in this embodiment, the MAMS system has 2 taggants that confirm placement of the pill into the mouth, and 2 taggants that will confirm the subject actually took the pill. By adding two taggants for each function, the reliability of the system will become much greater than if one was used for each. In addition, placing Tlightgray1 and Tlightgray2 inside the pill will increase the reliability of the system in terms of making the generation of their respective metabolites more reliable and efficient.

In Figure 52, two taggants (Tgray and Tblack) are both placed on the surface of an isotopic-labeled therapeutic agent, *A. Note: In previous embodiments (Figures 49-51), A was not labeled with non-ordinary isotopes. The **EDIMs** are **Tgray, Tblack,** and metabolite of *A, ***A1**. When placed in the mouth, two pill surface-derived EDIMs (Tgray and Tblack) are immediately liberated and will activate a sensor when exhaled into shortly (detection of Tgray and Tblack). Later, after ingesting the pill, *A enters the GIT, absorbed in the blood, and then metabolized to *A1, which will appear in the breath and activate the breath sensor (detection of *A1). Different surface taggants could be used to label different doses of *A. This embodiment provides immediate notification of pill ingestion and confirmation of pill ingestion. The chance of interference of EDIM detection to document the pill was placed in the mouth by various factors (e.g., diet, metabolism, disease) is very low if Tgray and Tblack are simultaneously detected in breath. *A provides confirmation that the pill was actually ingested. In addition, if a medication can become "self reporting" in terms of their metabolism, it would markedly improve drug safety.

In Figure 53, two taggants (Tgray and Tblack) are both placed on the surface of an isotopic-labeled therapeutic agent, *A. Note; in previous embodiments (Figures 49-51), A was not labeled with non-ordinary isotopes. The **EDIM** are **Tgray, Tblack,** and metabolite of *A, ***A1**. The only difference between this embodiment and that of Figure 52 is the mass of isotopic-labeled active therapeutic drug in the pill. In the example shown, only 0.1% of the mass of the active therapeutic drug located within the capsule contains non-ordinary isotopes. Given the signal-to-noise ratio that isotopic labeled EDIM (derived from *A) provides, there is no reason to label the majority of the mass of A. The preferred amount of *A in the pill is the least amount of *A that stills provides an adequate *A-based EDIM signal and thus an effective MAMS.

In Figure 54, two taggants (Tdarkgray and Tblack) located on the surface and one taggant (Tlightgray) is placed inside the pill in a manner that makes it physically distinct from the isotope-labeled active therapeutic agent, *A. The **EDIMs** are **Tdarkgray, Tblack,** and a metabolite of Tlightgray, **T1lightgray,** and a metabolite of *A, ***A1**. When placed in mouth, two pill surface-derived EDIMs (Tdarkgray and Tblack) are immediately liberated and will activate a sensor when exhaled into shortly (detection of Tdarkgray and Tblack) after ingesting the pill; later, when Tlightgray enters the GIT and is absorbed into the blood, it is metabolized to T1lightgray that will appear in the breath and activate the sensor when exhaled into (detection of T1lightgray). The only difference between this embodiment and that of Figure 52 is the addition of a taggant, Tlightgray, inside the pill. Likewise, Tlightgray could be placed on the pill surface, preferably in a more protected manner than Tdarkgray and Tblack. Since using *A1 alone is problematic for assessing drug adherence (see Figure 52), Tlightgray will address this issue. In fact, in this embodiment, Tlightgray serves not only to indicate that the pill contents entered the blood (definitive adherence) but also provides a critical comparator required to properly assess the metabolism of *A to *A1. The latter relates to correcting for changes in gastric emptying and/or drug absorption. Its placement inside the pill (versus on the surface) makes Tlightgray a more efficient source of T1lightgray (more reliable delivery to the GIT and blood entry), and hence improves the quality of MAMS. The active therapeutic drug will "self report" its metabolism via *A1 EDIM breath concentrations and adjustments will be made using Tlightgray.

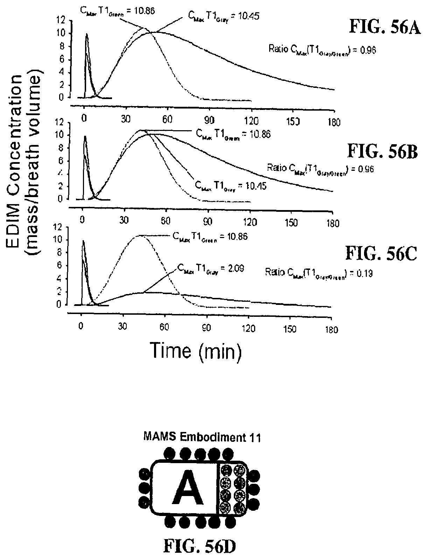

In Figure 55, two taggants (Tdarkgray, Tblack) on the pill surface and two taggants (Tlightgray1, Tlightgray) within the pill containing the active therapeutic agent, A. In this embodiment, A does not contain a non-ordinary isotope. The **EDIMs** are **Tdarkgray, Tblack,** a metabolite of Tlightgray1, **T1lightgray1,** and a metabolite of Tlightgray2, **T1lightgray2.** When placed in mouth, two pill surface-derived EDIMs (Tdarkgray and Tblack) are immediately liberated and will activate a sensor when exhaled into shortly (detection of Tdarkgray and Tblack) after ingesting the pill; later when Tlightgray1 and Tlightgray2 enter the GIT and are absorbed into the blood, it is metabolized to T1lightgray1 and T1lightgray2 that will appear in the breath and activate the sensor when exhaled into (detection of T1lightgray1 and T1lightgray2). The only difference between this embodiment and that of Figure 54 is the addition of a taggant, Tlightgray2, inside the pill, alongside Tlightgray1. In this embodiment, like that of Figure 54, Tlightgray is metabolized by a different enzyme than that for A, preferably a high capacity, rapidly acting blood-based enzyme (e.g., butyrylcholinesterase). In contrast, Tlightgray2 is metabolized by the same major enzyme, termed E, as that of A. In other words, Tlightgray2 (via conversion by E to T1lightgray2) will be used as a probe to continuously assess the metabolism of A to A1. In some cases, excellent probes exist that can predict the metabolism of key therapeutic agents. This approach has many advantages: 1) no requirement to isotopically label A, 2) not limited by mass or half life of A in terms of detecting breath A1 to assess metabolism of A, and 3) probes exist that accurately predict metabolism of important drugs. To illustrate this concept, the desmethylation of the antipsychotic olanzapine (Figure 45) by CYP-1A2 to liberate formaldehyde is well predicted by caffeine (Figure 46) when used as an enzyme probe. Olanzapine is potent (typical dose = 10 mg PO QD). Given its long half life and low dose, olanzapine won't generate high breath A1 concentrations. In contrast, a much greater mass (typical dose is hundreds of mg per day orally) of the GRAS-food additive caffeine can be safely given to humans, which will markedly increase the signal-to-noise ratio (e.g., levels of deuterated breath formaldehyde) and accurately predict the metabolism of olanzapine,. TGreen functions independent of TGray and is still required to ensure the pill was actually ingested (see Figure 52 for discussion) and to provide adequate corrections for GIT factors. Figure 56 illustrates how this system would work.

Figures 56A-C show the weekly EDIM concentration-time relations in a subject after swallowing a pill (having the architecture of MAMS-11) once per day over 3 weeks. Panel A, B and C illustrate the EDIM concentration-time relations at Day 7, Day 14, and Day 21, respectively, of therapy with active therapeutic drug A. To measure the EDIM concentrations, end tidal (alveolar values) is the phase of breath preferred, particularly for the more volatile EDIMs. At Day 7, Day 14, and Day 21, the subject regularly and reliably placed the pill in his/her mouth (i.e., CMax of Tdarkgray and CMax of Tblack were unchanged over the 3 weeks). Likewise, because CMax of T1lightgray1 did not vary between weeks, it appears this subject has minimal variation in his/her gastric emptying/absorption of the pill. During the first two weeks of therapy, the metabolism of Tlightgray2 (taggant substrate that is metabolized by the same enzyme that metabolizes A to A1) is stable. However, at Day 21 the CMax ratio of T1lightgray2:T1lightgray1 plummeted by 5-fold (0.96 to 0.19), indicating the metabolism of Tlightgray2 was likely to be severely reduced. Because this ratio takes into account Tlightgray1, this reduction in Tlightgray2 cannot be attributed to alterations in GIT function (gastric emptying, absorption). It was later learned the subject was placed on a 5th medication that caused a DDI (inhibited the CYP450 enzyme that metabolized both Tlightgray2 and A). Note: Other parameters that could be used in the analysis include but are not limited to area under the concentration-time curve (AUC), rate of increase and/or decrease of EDIM concentrations, and time to maximum concentration (TMax). In embodiments such as this embodiment where FMetab is assessed, the preferred measure of EDIMs used to assess drug metabolism is quantitative; assessment of surface EDIM markers to indicate placement of the pill in the mouth can be semiquantitative or even qualitative.

In Figure 56D, two taggants (Tdarkgray, Tblack) on the pill surface and two taggants (Tlightgray1, Tlightgray2) within the pill containing the active therapeutic agent, A. In this embodiment, A does not contain a non-ordinary isotope. The **EDIMs** are **Tdarkgray, Tblack,** a metabolite of Tlightgray1, **T1lightgray1,** and a metabolite of Tlightgray2, **Tllightgray2.** When placed in mouth, two pill surface-derived EDIMs (Tdarkgray and Tblack) are immediately liberated and will activate a sensor when exhaled into shortly (detection of Tdarkgray and Tblack) after ingesting the pill; later when Tlightgray1 and Tlightgray2 enter the GIT and are absorbed into the blood, it is metabolized to T1lightgray1 and T1lightgray2 that will appear in the breath and activate the sensor when exhaled into (detection of T1lightgray1 and T1lightgray2). Please see Figure 55 for a description of the function of T1lightgray1 and T1lightgray2.

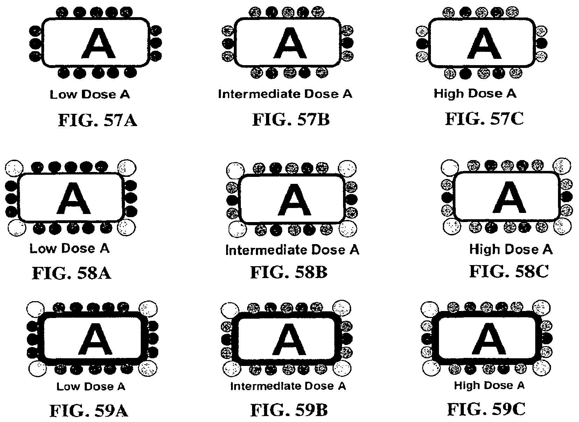

In Figures 57A-C, three sets of dual taggants located on the pill surface containing the active therapeutic agent, A are used to label three different doses of A. The three sets of surface taggants include a) Tdarkgray -Tblack (low dose A), b) Tlightgray -Tdarkgray2 (intermediate dose A), and c) Tlightgray2 -Tdarkgray3 (high dose A). The surface taggants could be solid-based and/or liquids contained in biodegradable capsules adhered to the surface of A. The **EDIMs** are **Tdarkgray -Tblack** (low dose A); **Tlightgray -Tdarkgray2** (intermediate dose A); **Tlightgray2 -Tdarkgray3** (high dose A). When placed in the mouth, two pill surface-derived EDIMs for each dose form are immediately liberated and will activate a sensor (indicates detection of the two taggants for each dose) when exhaled into. In this embodiment, immediate notification of pill ingestion and simplicity of system are provided. Further, chance of interference of EDIM detection by various factors (e.g., diet, metabolism, disease) is very low using dual system of surface taggants, particularly when they are simultaneously detected in breath.

In Figures 58A-C, three different dose forms of a given active therapeutic agent, A, are surfaced labeled by using different markers, consisting but not limited to a total of seven taggants (Twhite, Tdarkgray, Tblack, Tlightgray, Tdarkgray2, Tlightgray2, Tdarkgray2) on the pill surface containing the active therapeutic agent, A. In this embodiment, one taggant (Twhite) is used to label the active therapeutic agent, which has multiple dose forms. The other six taggants are used to label the dose; in this embodiment, two unique surface taggants are used to label the dose form: 1) low dose: Tdarkgray and Tblack; 2) intermediate dose: Tlightgray and Tdarkgray2; and 3) high dose: Tlightgray2 and Tdarkgray3. The surface taggants could be solid-based and/or liquids contained in biodegradable capsules adhered to the surface of A. The **EDIMs** are 1) low dose: **Twhite, Tdarkgray** and **Tblack;** 2) intermediate dose: **Twhite, Tlightgray** and **Tdarkgray2;** and 3) high dose: **Twhite, Tlightgray2** and **Tdarkgray3.** When a given dose of active therapeutic agent A is placed in the mouth, three pill surface-derived EDIMs are immediately liberated and will activate a sensor when exhaled into, indicating placement of drug A and a specific dose of drug A into the mouth. In this embodiment, immediate notification of pill ingestion and simplicity of system is provided. Further, chance of interference of EDIM detection in breath by various factors (e.g., diet, metabolism, disease) is very low with multiple surface taggant system, particularly if they are simultaneously detected in breath.

In Figures 59A-C, three different dose forms of a given active therapeutic agent, A, are surfaced labeled by using different surface markers, consisting of seven taggants (Twhite, Tdarkgray, Tblack, Tlightgray, Tdarkgray2, Tlightgray2, Tdarkgray2) "loosely" attached and one taggant (Tdarkoutline) firmly adherent to the pill surface containing the active therapeutic agent, A. One taggant (Twhite) is used to label the active therapeutic agent, which has multiple dose forms. Another taggant (Tdarkoutline), via enzymatic (preferably a blood-based enzyme) generation of a metabolite, T1darkoutline, is used to guarantee the pill contents entered the blood of the subject following GIT absorption. The remaining six taggants are used to label the dose. In this embodiment, two unique surface taggants are used to label the dose form: 1) low dose: Tdarkgray and Tblack; 2) intermediate dose: Tlightgray and Tdarkgray2; and 3) high dose: Tlightgray2 and Tdarkgray3.. In this embodiment, 7 surface taggants (Twhite, Tdarkgray, Tblack, Tlightgray, Tdarkgray2, Tlightgray2, Tdarkgray3) are designed to be easily released in the mouth, whereas the one surface tightly adherent taggant (Tdarkoutline) is designed to be preferentially released in the stomach or more distal GIT locations (e.g., duodenum). These taggants could be solid-based and/or liquids contained in biodegradable capsules attached, either loosely and/or tightly, to the surface of A. The **EDIMs** are **T1darkoutline** plus 1) low dose: **Twhite, Tdarkgray** and **Tblack;** 2) intermediate dose: **Twhite, Tlightgray** and **Tdarkgray2;** and 3) high dose: **Twhite, Tlightgray2** and **Tdarkgray3.** When a given dose of active therapeutic agent A is placed in the mouth, three pill surface-derived EDIMs are immediately liberated and will activate a sensor when exhaled into, indicating placement of drug A and a specific dose of drug A into the mouth. This embodiment is the same as that of Figure 58 except a taggant Tdarkoutline has been added that generates T1darkoutline, which confirms the pill contents entered the blood and the pill was actually ingested. In the preferred embodiment, Tdarkoutline is firmly attached to the surface of the active therapeutic agent (i.e., does not dislodge or be released in the mouth) or integrated into the gel matrix of a hard gel capsule and therefore neither alters the matrix of A nor the require a separate compartment within the a pill (which still keeps Tdarkoutline apart from the matrix of A).

Below is a succinct description of the components and features of various NICE systems and the rationale behind them.

**Sensors:** To create NICE-type therapeutic agents, the measurement of various entities, either from the active drug and/or associated taggants *per se* or from their respective metabolites, will be measured using sensing technology, preferred but not limited to being portable point-of-use devices. The types of sensors were previously disclosed in the above patents (Section A), but include and are not limited to the various types of infrared spectroscopy (gas or liquid based) with or without GC or mGC, mass spectroscopy (SIFT, GC, liquid), infrared, Raman, GC-MS, and neutron diffraction. The sensors would use various biological media including breath, blood, urine etc In a preferred embodiment a sensor could use two types of sensing technologies (e.g., IR and mGC-CMOS), which would in turn provide a much greater level of discrimination between molecular entities (drugs and taggants) if stable isotope labeling was combined with discrimination of say alcohols.

**Key Characteristics of NICE:** Multiple types of NICE-type therapeutic agents are created by assembling different combinations of different types of design elements into the system (Figures 49-59). These elements provide a chemical framework whereby the system can optimally (reliably, reproducibly, and accurately) assess F_{Freq}, F_{Dose}, and/or F_{Metab}, and correct for factors that would confound interpretation and function of the NICE system. These factors include: 1) correction for variable gastric emptying (e.g., slowing of gastrokinesis due to stress, consumption of fatty meals, or drugs) and/or absorption that can alter oral drug pharmacokinetics such as area under the concentration-time curve (AUC), time to maximal concentration (Tₘₐₓ) and maximal concentration (Cₘₐₓ), 2) detection and correction for impaired enzyme function (e.g., secondary to genetic polymorphisms, drug-drug interactions (DDIs), pathophysiological disturbances) that may mask administration (false negative) of drug ingestion when it was actually imbibed, if the exhaled drug ingestion marker(s) (EDIM) is generated via that particular enzyme, 3) provide a high degree of discrimination of detecting volatile (or semi-volatile, and even non-volatile) markers in the breath against a background of endogenous production of similar or identical substance or dietary intake of substances in foods/drinks (e.g. effects of fatty meals on bioavailability, generation of volatile markers used in the NICE system), and 4) generate markers, termed exhaled drug ingestion markers (EDIMs) in the breath, which are suitable for NICE systems (e.g., duration neither too short nor too long; reliably appears in the breath). Indeed, the comparator, as described in Option 2 of Section B3, not only would ensure that the metabolism of the active therapeutic drug A was being metabolized normally, but also could be used as an index of gastric emptying in a variety of clinical settings.

Features of these NICE elements include but are not limited to the following:
- the active therapeutic drug could be a generic drug, patented drug, or other type of pharmaceutic.
- the taggant(s) could be associated with A by surface coating, physically locating them in different compartments of a capsule/pill, or integrating the taggant into the excipient matrix of a pill or capsule. The preferred embodiment is to place the taggant in a manner that does not alter the FDA-approved pill matrix (e.g., taggant integrated into the matrix of a hard gel capsule that contains the API inside it).
- A taggant should be added to correct for changes in gastroesophageal emptying, absorption, metabolic incompetence of specific enzymes.
- The dose of an active pharmaceutical drug could be determined using the NICE system by associating different doses of the active therapeutic agent with the following strategies: a) incorporate different isotopes on various parts of the active therapeutic agent's and/or taggants' molecular structures in the NICE system, preferably on those that liberate volatile (or semi-volatile) metabolic fragments upon enzyme degradation; this dose not exclude non-ordinary isotopic labeling of larger, non-volatile fragments of the parent compound; b) incorporate variable extents of a given isotopic label (e.g., deuterium) on the active therapeutic agent and/or taggants in the NICE system, c) incorporate combinations of a and b in the NICE system, and/or d) incorporate different doses of a given taggant with or without isotopic labeling place.
- The taggants could be any Class 1, 2 and/or 3 drugs (see Section A) including but not limited to new chemical entities or GRAS-type compounds, which may or may not be labeled with non-ordinary isotopes (see Table 2).
- The isotopic labels could be located on one or more locations of active drug(s) or taggant(s). The active therapeutic agent and associated taggant(s), and their respective metabolites may or may not utilize isotopic labels in the NICE system.
- A molecule, either the active therapeutic agent (A) and/or taggant (T), could contain a single or different types of stable isotopic labels (see Table 2).
- The enzymes used to degrade the compound(s) to generate various EDIMs may or may not be the same as the primary enzyme used to degrade the active therapeutic agent, A. The enzymes involved in the NICE system could include but are not limited to: a) oxidative metabolism involving CYP450, including but not limited to important isoforms for drug metabolism such as CYP-3A4 and CYP-2D6, or those impacted by genetic polymorphisms (CYP-2D6, 2C19, 2C9), b) VKORC1 in the setting of warfarin therapy, c) esterases including but not limited to pseudocholinesterase, carboxylesterases, PON1, and acetylcholinesterase, etc.), d) dehydrogenases (alcohol and aldehyde), and e) enzymes not listed above but listed in the patent (Table 1).
- The enzyme substrates used in the NICE system, which entail the active therapeutic agent, drug salt (S), excipients (E) and/or taggants (if present), when acted upon by CYP450, esterases, and/or other enzymes, will generate volatile (or semivolatile, nonvolatile) markers that appear in the breath, termed the EDIMs. These breath markers, which themselves can be Class 1, 2 and/or 3 drugs or be derived by metabolism of Class 1, 2 and/or 3 drugs, will include but are not limited to alcohols, aldehydes, ketones, and acids. Section B.2 lists all relevant chemicals. Alternately, in some embodiments of the invention, the active therapeutic agent and/or the taggant(s) itself may be detected in the breath.
- The enzyme substrates (active therapeutic agents and/or taggants) of the NICE system could be in physical state of solid, liquid or gas. Solid or liquids having lower volatility are the preferred embodiments in the NICE system for the active therapeutic drug and taggants.
- A subject may blow (preferentially once but be more than once in a given session) into the device to rapidly check for F_{Freq}F_{Dose}F_{Metab}, or at a less frequent basis (e.g., once per week or month) blow repeatedly into the device at fixed time intervals over a longer period of time (e.g., 1-3 hrs) to get a complete breath concentration-time relationship to fully assess metabolic competence.
- The sensor of the NICE system may or may not be linked to a biometric (e.g., fingerprint, retinal scan)

Another means to produce drugs for the above applications, which would be both economically feasible and straightforward in terms of detection, includes modifying (labeling) known compounds (Class I through III type agents, see above) with a stable isotope (see Table 1 for examples using in biology) including, but not limited to, deuterium (heavy water, or heavy hydrogen). In preferred embodiment, the isotopic label would stable (non-radioactive), and be deuterium. Deuterium is a stable, non-radioactive isotope of hydrogen that is found naturally, which contains 1 proton and 1 neutron electron. The number of protons plus neutrons is called the atomic mass number. An isotope is an atom with the same number of electrons and protons, but with a different number of neutrons. In other words, atoms with the same atomic number but different atomic weights are called isotopes. Because specific types of isotopes have an identical number of electrons, they belong to the same element and behave almost the same in chemical reactions. Therefore, in medicine isotopes such as deuterium have been used to label biologically important molecules in metabolic studies as a non-radioactive isotopic tracer because chemically it behaves very similarly to hydrogen, is essentially non-toxic, and can be readily distinguished from hydrogen using infrared (IR) or mass spectrometry. Although other non-radioactive stable isotopes (see Table 1) such as carbon (e.g., ¹³C) could be used in these technologies, deuterium is strongly preferred because it can be more readily detected and discriminated from endogenous molecules containing ordinary hydrogen using inexpensive, portable, point-of-care and point-of-use sensing technologies such as IR. Either a deuterated parent molecule containing the deuterium label and/or a key volatile (or semi-volatile) metabolite(s) of the parent molecule (generated via enzyme metabolism) containing the deuterium would be detected in the breath.

Deuterium, depending upon the class of molecules they are placed on, the number of deuterations on a molecule, and their proximity to various bond types (e.g., amine, sulfhydryl, aromatic, etc.) on the molecule, can provide various types of molecular entities with unique analytical "signatures" in various biological media, including but not limited to breath, blood, urine, sweat or saliva. Various analytical techniques such as IR or mass spectroscopy can be used to not only distinguish deuterated parent compounds from their deuterated metabolites (both in the gas and/or liquid states), but can also easily discriminate deuterated molecules from those identical natural compounds containing ordinary hydrogen (e.g., ethanol versus deuterated alcohol; aldehyde versus deuterated aldehyde; methanol versus deuterated methanol). The use of deuterium can be applied to all of the inventions listed above. Its use will reduce the need or even eliminate the step of obtaining baseline breath samples, as well as markedly simplify (or even eliminate) the FDA regulatory process for new drugs allowing for faster time to market with inexpensive and reliable technology.

Deuterated compounds are generally regarded as nontoxic and of having the same (or very similar) pharmacodynamic (PD) and pharmacokinetic (PK; ADME) properties as their undeuterated parent compounds. Further, deuteration can be applied to several new related inventions (which can also be practiced with the more conventional Class 1 through 3 agents disclosed for medical diagnostic applications). They are:
- "New Intelligent Chemical Entity" (NICE)-type therapeutic agents for medication adherence monitoring. Three different types of NICE-type agents exist for medication adherence in this application: **1**) the parent therapeutic agent being used to treat a medical disorder is labeled with deuterium and upon metabolism (e.g., via enzymatic action) will generate a volatile (or semi-volatile) marker in the breath containing the deuterium label, **2**) the therapeutic agent is not labeled *per se* with deuterium but upon metabolism (e.g., via enzymatic action) will generate a volatile or semi-volatile (not deuterated) marker that can be detected in the breath, and **3**) the therapeutic agent is neither labeled with deuterium nor generates a measurable volatile (or semi-volatile) marker in the breath, but is rather associated with a taggant (that may be either labeled or unlabeled with deuterium), which in turn will generate a marker in the breath that is easily measurable. The taggant can be part of the excipient matrix/salt of the therapeutic drug, can be coated onto the surface of the therapeutic drug, or be physically separate from the therapeutic drug. The preferred taggant embodiment is the latter case where the excipient matrix of the active therapeutic drug is not altered by the presence of the taggant.
- NICE-type drugs to assess enzyme competency. Three different types of NICE compounds exist for assessing enzyme competency in this application: **1**) the parent therapeutic agent would contain a deuterated label and would itself "report" whether the individual taking the medication is competent for the enzyme required to metabolize itself by measuring a deuterated metabolite of the parent drug in the breath, and **2**) the parent therapeutic agent would not contain a deuterated label but would still itself "report" whether the individual taking the medication is competent for the enzyme required to metabolize itself by measuring a metabolite (non-deuterated) of the parent drug in the breath, and **3**) the taggant, either labeled or not labeled with non-ordinary isotopes, associated with the active therapeutic agent A would "report" whether the individual taking the drug is competent for the enzyme required to metabolize A. NICE-type agents used for enzyme competency could have additional taggant(s) added, besides the ones mentioned above, which would serve a number of important roles: a) an enzyme calibrator by being a substrate for the same or different enzyme as the one degrading the therapeutic agent, and b) an index of gastric emptying to correct for the effect of varied gastric clearance on enzyme competency results.
- The means to synthesize taggants or modified pharmaceutics is disclosed in detail in the prior patent applications, as are lists of GRAS and other compounds which can be used to monitor adherence, enzyme competency and drug diversion. As previously disclosed, most medications are metabolized by the P450 enzyme system, with the CYP2D6 and CYP3A4 isoforms accounting for about 83% of drug metabolism (3A4 - 50% and 2D6 - 33%). With so many drugs metabolized by a limited number of enzymes, there is often competition for metabolism. Likewise, genetics can alter the function of many of these CYP enzymes (e.g., CYP 2D6, CYP 2C9, CYP 2C19). Also, certain drugs can induce P450 enzymes and actually reduce blood concentrations of other drugs. With the average individual over 65 years of age taking 4 or more medications, it is not surprising that there are frequent adverse drug reactions (ADRs). It is estimated that over 700,000 serious reactions and deaths result from DDIs.
- Although these drugs can be potentially given via various routes (oral, intravenous, sublingual, rectal, intramuscular, inhalation, subcutaneous, intrasynovial, intracardiac, intrathecal, intratracheal, buccal, eye, nasal, ear, rectum, vaginal, urethral, transdermal/topical), the preferred administration routes will be oral and sublingual.
- Combining adherence monitoring and enzyme competency reporting in a NICE-type therapeutic agent is novel, particularly when it is done in a continuous basis and is an intrinsic part of the therapeutic agent (EDIM is generated from the therapeutic agent or from a taggant physically associated with the therapeutic agent).
- The dual function of a NICE-type therapeutic agent could come from one specific area of the parent molecule or from more than one area of the molecule. For example, 1 specific metabolic fragment of a NICE would indicate adherence, where another fragment of a NICE would indicate it is being metabolized properly or improperly. In another embodiment, a specific fragment of a NICE would indicate both adherence and metabolic functions.
- If a therapeutic agent undergoes metabolism by multiple enzymes, multiple taggants (either labeled or not labeled with stable isotopes), could be added to monitor its metabolism. For example, lets say a chemical entity termed X undergoes P450 oxidation metabolism via CYP-3A4 and CYP-2D6. Two taggants, termed T_{3A4} and T_{2D6}, either labeled with or without an isotope (e.g., deuterium), which are known substrates for CYP-3A4 and CYP-2D6, respectively, would be physically associated with X. Volatile (or semi-volatile) fragments from T_{3A4} and T_{2D6} that appear in body fluids such as the breath, could be used to quantitate metabolism via the different pathways of X.
- To document MAM, if a taggant approach is used, then the taggant may be metabolized by a different enzyme system than the therapeutic agent. This may be advantageous since alterations in the enzyme that degrades the active therapeutic agent A may diminish the ability to use it as a MAMs marker, if it is not being metabolized property. To get around this limitation and to eliminate changes in esophageal/gastric motility with oral pill ingestion, a comparator taggant could be added, which will document that the components of the pill are being delivered to the blood and liver, and we can normalize the AUC and/or C_{Max} to document things (Figures 54-56).
- The isotope label could be incorporated into the active therapeutic agent in two ways: 1) 100% of the active therapeutic agent contains the isotopic label (Figure 52), or 2) only a fraction of the active therapeutic agent (Figure 53).

In this patent application, technologies are described that catalyze a new strategy in drug design and drug research and development - "smart" (self monitoring and reporting therapeutics) drugs. By markedly reducing the incidence of ADRs and ensuring medication adherence, NICE-type agents will markedly improve both drug safety and efficacy while simultaneously reducing health care costs. Unique features about NICE-type drugs are that the "reporter" (EDIM) is a stable isotopic label entity (e.g., deuterium) as well the disclosure of new medical uses for exhaled breath. Stable isotopes present several advantages over all of the previously disclosed taggants and detection devices. First, a stable isotope such as deuterium should be regarded by the FDA to be safe and having minimal-to-no effect on PK/PD. Isotope (e.g., deuterated)-labeled chemicals are readily available, mostly for calibration of analytical equipment used for therapeutic drug monitoring and synthesis of deuterated analogues is straightforward and inexpensive. Further, deuteration of a compound changes the IR spectrum (liquid and gas phases) so that the deuterated analog can be easily distinguished from the parent compound.

The technology outlined in this invention will not only allow monitoring of medication adherence and enzymatic (metabolic) competence on a continuous on-going basis to make therapies (acute and chronic) drugs more safe and efficacious, but can be readily adapted to address other areas of national and international importance including drug counterfeiting, drug diversion and therapeutic drug monitoring (TDM) using cost effective technologies.

All patents, patent applications, and publications referred to or cited herein are incorporated by reference in their entirety, including all figures and tables, to the extent they are not inconsistent with the explicit teachings of this specification.

It should be understood that the examples and embodiments described herein are for illustrative purposes only and that various modifications or changes in light thereof will be suggested to persons skilled in the art and are to be included within the spirit and purview of this application.

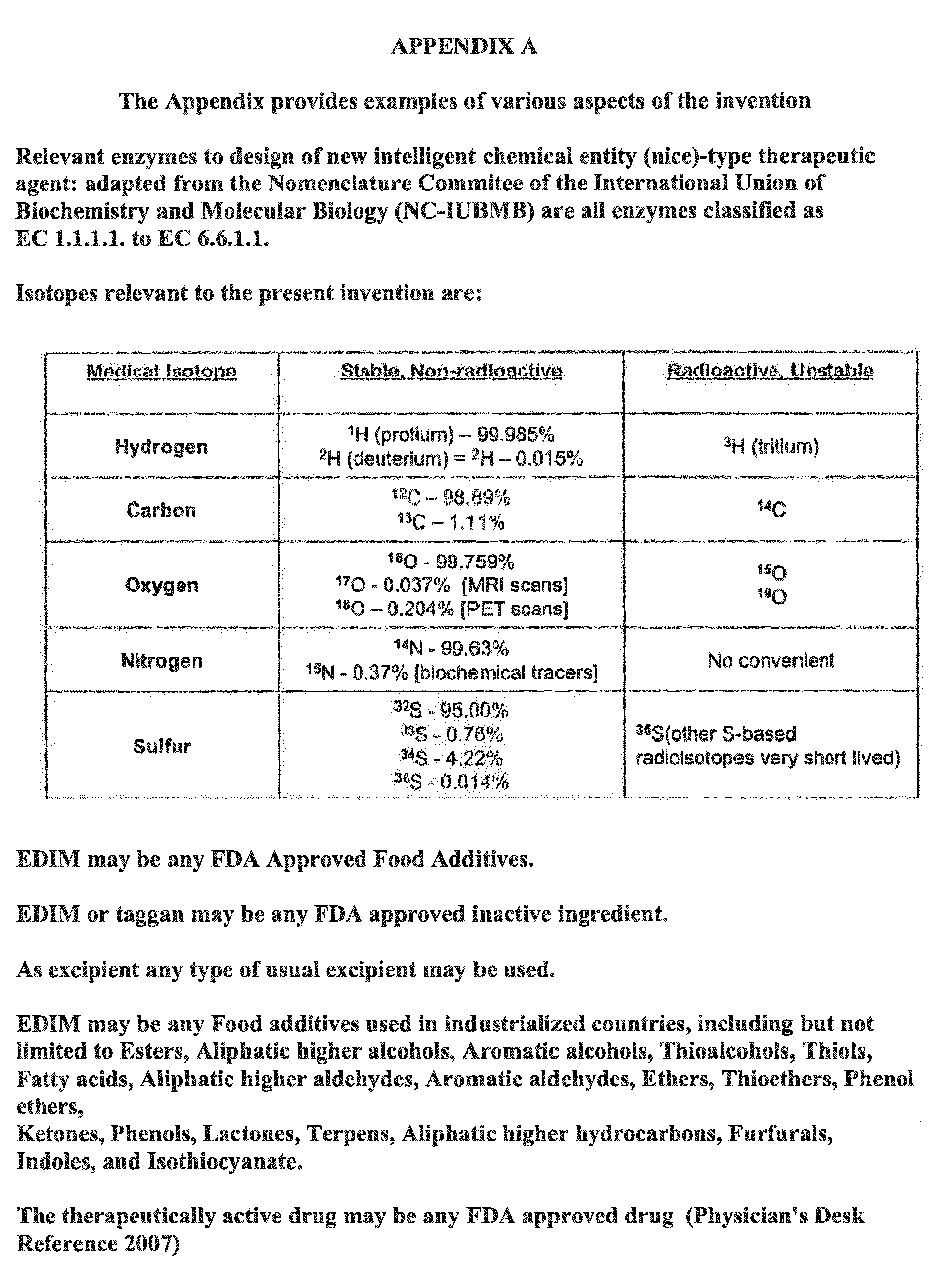

## Claims

1. A medication pill comprising:
a therapeutic drug; and
a first isotopically-labelled exhaled drug ingestion marker (EDIM), or a taggant that comprises the first isotopically-labelled EDIM or metabolizes to form the first isotopically-labelled EDIM; and
a second isotopically-labelled EDIM, or a taggant that comprises the second isotopically-labelled EDIM or metabolizes to form the second isotopically-labelled EDIM.

2. The medication of claim 1, wherein each isotopically-labelled EDIM or taggant is a generally regarded as safe (GRAS) compound.

3. The medication of claim 1, wherein at least one isotopically-labelled EDIM comprises deuterium.

4. The medication of claim 1, wherein the first isotopically-labelled EDIM, or the taggant that comprises the first isotopically-labelled EDIM or metabolizes to form the first isotopically-labelled EDIM, is coated onto a surface of the pill; and
the second isotopically-labelled EDIM, or the taggant that comprises the second isotopically-labelled EDIM or metabolizes to form the second isotopically-labelled EDIM, is also coated onto the surface of the pill.

5. The medication of claim 1, wherein the first isotopically-labelled EDIM, or the taggant that comprises the first isotopically-labelled EDIM or metabolizes to form the first isotopically-labelled EDIM, is coated onto a surface of the pill; and
the second isotopically-labelled EDIM, or the taggant that comprises the second isotopically-labelled EDIM or metabolizes to form the second isotopically-labelled EDIM, is present inside the pill.

6. The medication of claim 1, wherein the medication further comprises a third isotopically-labelled EDIM, or a taggant that comprises the third isotopically-labelled EDIM or metabolizes to form the third isotopically-labelled EDIM.

7. The medication of claim 6, wherein the first isotopically-labelled EDIM, or the taggant that comprises the first isotopically-labelled EDIM or metabolizes to form the first isotopically-labelled EDIM, is coated onto a surface of the pill;
the third isotopically-labelled EDIM, or the taggant that comprises the third isotopically-labelled EDIM or metabolizes to form the third isotopically-labelled EDIM, is coated onto the surface of the pill; and
the second isotopically-labelled EDIM, or the taggant that comprises the second isotopically-labelled EDIM or metabolizes to form the second isotopically-labelled EDIM, is present inside the pill.

8. The medication of claim 1, wherein the therapeutic drug is also isotopically-labelled.
